# EUROPEAN PATENT SPECIFICATION

(11) **EP 1 919 938 B1**
(45) Date of publication and mention of the grant of the patent: **06.06.2012**
(21) Application number: 06791714.6
(22) Date of filing: 29.08.2006
(51) Int. Cl.: C07K 14/435, C12N 15/12

(54) **MODIFIED SPIDER SILK PROTEINS**
MODIFIZIERTE SPINNENSEIDEPROTEINE
PROTÉINES DE SOIE ARACHNÉENNE MODIFIÉES

(30) Priority: 29.08.2005 US 712095 P
(43) Date of publication of application: 14.05.2008
(73) Proprietor: AMSilk GmbH, 82152 Planegg/Martinsried (DE)
(72) Inventor: SCHEIBEL, Thomas, 81673 Muenchen (DE)
(74) Representative: Zwicker, Jörk
(86) International application number: PCT/EP2006/008452
(87) International publication number: WO 2007/025719

(56) References cited:
- EP-A2- 1 413 585
- WO-A-03/057727
- WO-A2-2004/016651
- SCHEIBEL T: "Spider silks: Recombinant synthesis, assembly, spinning, and engineering of synthetic proteins" MICROBIAL CELL FACTORIES 16 NOV 2004 UNITED KINGDOM, vol. 3, 16 November 2004 (2004-11-16), page 10p, XP002407679 ISSN: 1475-2859
- ZHOU YINGTING ET AL: "Genetically directed synthesis and spectroscopic analysis of a protein polymer derived from a flagelliform silk sequence" BIOMACROMOLECULES, vol. 2, no. 1, April 2001 (2001-04), pages 111-125, XP002407680 ISSN: 1525-7797
- HINMAN M B ET AL: "Synthetic spider silk: a modular fiber" TRENDS IN BIOTECHNOLOGY, ELSEVIER, AMSTERDAM, NL, vol. 18, no. 9, 1 September 2000 (2000-09-01), pages 374-379, XP004214264 ISSN: 0167-7799
- HUEMMERICH D ET AL: "Primary structure elements of spider dragline silks and their contribution to protein solubility" BIOCHEMISTRY, AMERICAN CHEMICAL SOCIETY. EASTON, PA, US, vol. 43, no. 42, 26 October 2004 (2004-10-26), pages 13604-13612, XP002367100 ISSN: 0006-2960 cited in the application
- HUEMMERICH D ET AL: "Novel Assembly Properties of Recombinant Spider Dragline Silk Proteins" CURRENT BIOLOGY, CURRENT SCIENCE,, GB, vol. 14, no. 22, 23 November 2004 (2004-11-23), pages 2070-2074, XP004653121 ISSN: 0960-9822 cited in the application
- WONG PO FOO CHERYL ET AL: "Genetic engineering of fibrous proteins: Spider dragline silk and collagen" ADVANCED DRUG DELIVERY REVIEWS, AMSTERDAM, NL, vol. 54, no. 8, 18 October 2002 (2002-10-18), pages 1131-1143, XP002349316 ISSN: 0169-409X
- SCHEIBEL THOMAS ET AL: "Bidirectional amyloid fiber growth for a yeast prion determinant" CURRENT BIOLOGY, vol. 11, no. 5, 6 March 2001 (2001-03-06), pages 366-369, XP002407681 ISSN: 0960-9822

## Description

The present invention is directed to a modified spider silk protein. The invention further pertains to a nucleic acid sequence coding for said modified spider silk protein, a vector containing said sequences and host cells transformed with this vector. The invention furthermore is directed to a pharmaceutical or cosmetical composition containing a modified spider silk protein as defined herein and the use of said modified sequences in various fields, in particular in the fields of medicine, cosmetics and technical applications.

Spider silks are protein polymers that display extraordinary physical properties. Among the different types of spider silks, draglines are most intensely studied. Dragline silks are utilized by orb weaving spiders to build frame and radii of their nets and as lifelines that are permanently dragged behind. For these purposes high tensile strength and elasticity are required. The combination of such properties results in a toughness that is higher than that of most other known materials. Dragline silks are generally composed of two major proteins whose primary structures share a common repetitive architecture.

An orb web's capture spiral, in part composed of viscid silk formed by the flagelliform gland, which is therefore named flagelliform silk, is stretchy and can triple in length before breaking, but provides only half the tensile strength of dragline silk.

Variations of a single repeat unit, which can comprise up to 60 amino acids, are iterated several times to represent the largest part of a spider silk sequence. These repeat units comprehend a limited set of distinct amino acid motifs. One motif found in all dragline silk repeat units is a block of typically 6 - 9 alanine residues. In silk threads several polyalanine motifs form crystalline β-sheet stacks leading to tensile strength.

Glycine rich motifs such as GGX or GPGXX adopt flexible helical structures that connect crystalline regions and provide elasticity to the thread.

Silk assembly in vivo is a remarkable process. Spider dragline silk proteins are stored at concentrations up to 50 % (w/v) in the so-called major ampullate gland. Although a "dynamic loose helical structure" has been proposed for the proteins within the major ampullate gland more recent data suggests a random coil conformation for the proteins of the so called A-Zone, which represents the largest part of the gland. The highly concentrated protein solution forms the silk dope (spinning solution), which displays properties of a liquid crystal.

Thread assembly is initiated during a passage of the dope through the spinning duct accompanied by extraction of water, sodium and chloride. At the same time the concentrations of the more lyotropic ions potassium and phosphate are increased and the pH drops from 6.9 to 6.3. Assembly is finally triggered by mechanical stress, which is caused by pulling the thread out of the spider's abdomen.

For several purposes natural silk threads can not be used directly, but have to be dissolved and reassembled into other morphologies such as films, foams, spheres, nanofibrils, hydrogels and the like.

While some structural aspects of spider silk proteins have been unravelled, still little is known about the contribution of individual silk proteins and their primary structure elements to the assembly process. Comparative studies of the two major dragline silk proteins of the garden spider *Araneus diadematus,* ADF-3 and ADF-4, revealed that, although their amino acid sequences are rather similar, they display remarkably different solubility and assembly characteristics: While ADF-3 is soluble even at high concentrations, ADF-4 is virtually insoluble and self-assembles into filamentous structures under specific conditions (unpublished results).

Scientific and commercial interest initiated the investigation of industrial scale manufacturing of spider silk. Native spider silk production is impractical due to the cannibalism of spiders, and artificial production has encountered problems in achieving both sufficient protein yield and quality thread-assembly. Bacterial expression yielded low protein levels, likely caused by a different codon usage in bacteria and in spiders. Synthetic genes with a codon usage adapted to the expression host led to higher yields, but the proteins synthesized thereof showed different characteristics in comparison to native spider silks. Expression of partial dragline silk cDNAs in mammalian cell lines did yield silk proteins (e.g. ADF-3) that could be artificially spun into 'silken' threads, albeit as yet of inferior quality.

The inventors earlier developed systems for the recombinant production of spider silk proteins in *E*. *coli.* As an example, it is referred to WO 2006/008163 (claiming priority of US provisional application No. 60/590,196). In this expression system, single building blocks (= modules) can be varied freely and can thus be adapted to the requirements of the specific case. Modules of this type are disclosed also in Hümmerich, D., Helsen, C.W., Oschmann, J., Rudolph, R. & Scheibel, T. (2004): "Primary structure elements of dragline silks and their contribution to protein solubility and assembly, Biochemistry 43, 13604-13612".

Further, SCHEIBEL T. ("Spider silks: Recombinant synthesis, assembly, spinning, and engineering of synthetic proteins" MICROBIAL CELL FACTORIES, Vol. 3, November 16, 2004, pages 1 to 10) discloses synthetic proteins derived from spider silk.

ZHOU YINGTING ET AL. ("Genetically directed synthesis and spectroscopic analysis of a protein polymer derived from a flagelliform silk sequence" BIOMACROMOLECULES, Vol. 2, No. 1, April 2001, pages 111 to 125) relates to the investigation of the molecular mechanism underlying the elastomeric recovery of the capture spiral of arachnid webs. Therefore, a model polypeptide based upon the elastomeric repeat sequence of *Nephila clavipes* flagelliform silk protein has been synthesized using recombinant DNA techniques.

WO 2004/016651 A2 refers to the isolation and sequencing of a new silk polypeptide and includes vectors, transgenic plants and animals expressing DNA molecules encoding said silk polypeptide and methods for the production of said silk polypeptide.

WO 03/057727 A1 relates to a silk polypeptide comprising a plurality of repetitive units and a non-repetitive hydrophilic amino acid domain, a polynucleotide and a vector encoding said silk polypeptide, methods of expressing said silk polypeptide in host cells and transgenic animals, and methods of forming a biofilament comprised of said silk polypeptide.

One object of high relevance in particular for applications of spider silk proteins in the field of medicine is the covalent coupling of drugs, proteins, chemicals etc. to those spider silk proteins. However, up to now, no satisfying technique for coupling is known which allows on the one hand a coupling of those substances to spider silk proteins in a predetermined amount and, on the other hand, to predetermined locations within the spider silk protein.

Therefore, it is an object underlying the present invention to provide modified spider silk proteins which can be used for the targeted coupling of substances such as drugs, metals, polypeptides, polysaccharides, marker molecules, quantum dots, nucleic acids, lipids, etc. to these spider silk proteins. Such modified spider silk sequences can be used to carry and deliver a precise amount of those substances and wherein those substances are coupled in predetermined locations within the sequence of the spider silk protein.

This object is achieved by the subject-matter of the independent claims. Preferred embodiments are contained in the dependent claims.

Of major interest in this respect is the incorporation of amino acids in spider silk modules, which have a chemically specific amino acid side chain, in the present case a thiol group of cysteine or an amino group of lysine. None of the above mentioned modules of spider silk proteins, which have been described up to now, contains cysteine or lysine and thus, a specific mutagenesis of the respective nucleic acid sequences allows to incorporate the desired amino acids into the sequence of the modules in a controlled manner. Modules which have been modified in this way can be assembled to new constructs and therefore, by combination of single modules, also more than one chemical active agent or drug can be combined in one single construct.

Therefore, for the first time, a specific multiple coupling of reagents to recombinant spider silk proteins is feasible. Apart from the modification of the basic modules there is additionally the opportunity to couple chemically reactive amino acids by means of TAGs to the existing constructs in order to activate or modify same.

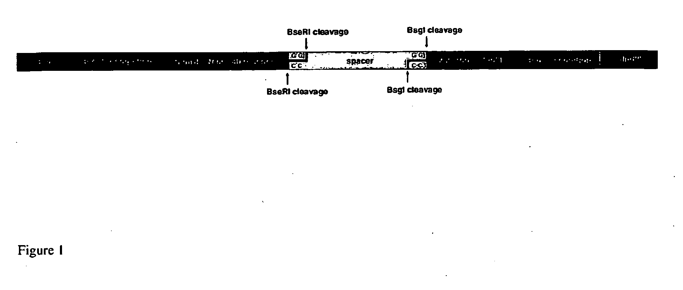

As mentioned above, the inventor himself generated an efficient production method of proteins similar to spider silk proteins and having characteristics, which can be specifically influenced by a cloning strategy which allows to assemble single DNA sequence modules in a controlled way to a synthetic gene (Hümmerich et al., 2004). The single modules are not spaced by foreign DNA sequences as it was the case in prior art cloning systems. In the presently used cloning system, as an example, the cloning vector pAZL (developed by the inventor) can be used, which contains a defined cloning cassette (figure 1). The most important elements of this cloning cassette are the recognition sequences for two restriction endonucleases (BseRI and BsgI), the restriction sites thereof being located 8 and 14 nucleotides, respectively, away from the respective recognition sequences. This allows the arrangement of translation start and stop codons and the integration of further restriction sites directly before or after the synthetic gene.

Between the restriction sites of BseRI and Bsgl a spacer region is present in the cloning cassette which will be replaced in the subsequent steps at first by single sequence modules and later by the synthetic gene. The arrangement of the single elements will be maintained in the subsequent steps (see Fig. 1).

The basis of the monomeric sequence modules are the genes ADF3 and ADF4 of the spider *Araneus diadematus* as well as the gene FLAG of the spider *Nephila clavipes.* Variations of the employed sequences of ADF3 and ADF4 are publicly available (available under the accession numbers U47855 and U47856). The first two genes (ADF3 and ADF4) are coding for proteins which are forming the dragline thread of the spider, the third is coding for a protein of the flagelliform silk. Based on the amino acid sequence of these proteins, several modules were designed:
Modul A: GPYGPGASAA AAAAGGYGPG SGQQ (SEQ ID NO: 1)
Modul C: GSSAAAAAAA ASGPGGYGPE NQGPSGPGGY GPGGP (SEQ ID NO: 3)
Modul Q: GPGQQGPGQQ GPGQQGPGQQ (SEQ ID NO: 2)
Modul K: GPGGAGGPYG PGGAGGPYGP GGAGGPY (SEQ ID NO: 4)
Modul sp: GGTTIIEDLD ITIDGADGPI TISEELTI (SEQ ID NO: 5)
Modul X: GGAGGAGGAG GSGGAGGS (SEQ ID NO: 6)
Modul Y: GPGGAGPGGY GPGGSGPGGY GPGGSGPGGY (SEQ ID NO: 7)

From these amino acid modules, synthetic spider silk protein constructs were assembled. These modules and the spider silk proteins derived therefrom form the starting material for the modified spider silk proteins according to the first aspect of they invention.

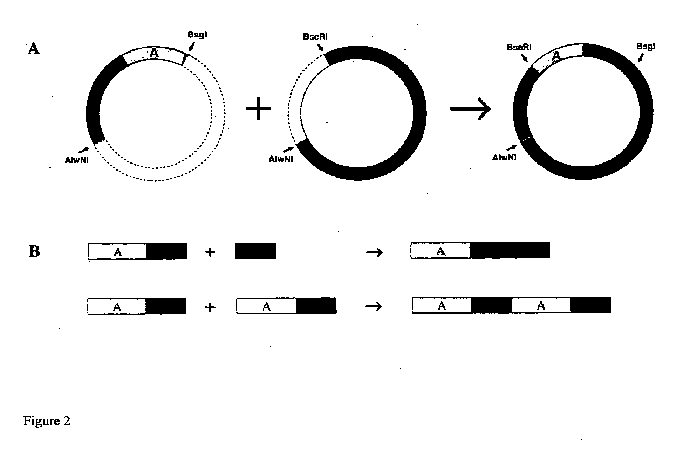

The structure of the cloning cassette allows an arbitrary assembly of two modules or module multimers in each case. See in this connection figure 2 which is showing an example of the multimerization of DNA-modules.

Herein a coupling system of several reagents to spider silk proteins is provided which makes it possible to perform different coupling reactions at the same time and without a high expenditure of work. This is a crucial requirement for potential industrial applications and production of coupled spider silk proteins.

Therefore, selected modules of spider silk proteins were modified in order to introduce amino acids with chemically distinct side chains in selected amino acid positions. The newly introduced amino acids are lysine and cysteine.

The present invention in particular is directed to the following aspects and embodiments:

In a first aspect, the present invention provides a modified spider silk protein comprising
(i) only one modified spider silk protein module according to SEQ ID NO: 1, SEQ ID NO: 3 or SEQ ID NO: 5 to SEQ ID NO: 7, or a variant thereof which differs in each case from said sequence by 1 amino acid substitution, 1 to 2 amino acid deletion(s), and/or 1 to 2 amino acid insertion(s), wherein one serine or glutamate in said spider silk protein module or variant thereof is replaced by one cysteine or lysine,
   and wherein optionally an amino terminal TAG according to SEQ ID NO: 20 to SEQ ID NO: 24 containing one lysine or cysteine among other amino acids, or a carboxyl terminal TAG according to SEQ ID NO: 25 to SEQ ID NO: 28 containing one lysine or cysteine among other amino acids is added to said module or variant thereof, or
(ii) one spider silk protein module according to SEQ ID NO: 1 to SEQ ID NO: 7, or a variant thereof which differs in each case from said sequence by 1 amino acid substitution, 1 to 2 amino acid deletion(s), and/or 1 to 2 amino acid insertion(s), wherein an amino terminal TAG according to SEQ ID NO: 20 to SEQ ID NO: 24 containing one lysine or cysteine among other amino acids, or a carboxyl terminal TAG according to SEQ ID NO: 25 to SEQ ID NO: 28 containing one lysine or cysteine among other amino acids is added to said module or variant thereof, and
wherein the modified spider silk protein does not comprise any further lysine or cysteine residues.

A modified spider silk protein according to the first aspect of the invention can be produced according to method of modifying a spider silk protein comprising the steps of:
a) providing a nucleic acid encoding a spider silk protein or a fragment thereof not containing lysine or cysteine residues;
b) replacing nucleic acids encoding one or more amino acids in said spider silk protein by a lysine or cysteine encoding nucleic acid sequence, and/or adding a nucleic acid sequence containing nucleic acids encoding lysine and/or cysteine to said sequence;
c) expressing the modified nucleic acid sequence obtained in b) in a suitable host, and
d) recovering the expressed modified spider silk protein.

The above method is the most efficient way of producing the modified spider silk proteins of the present invention. However, it is also possible to produce the same for example by providing a spider silk protein or a fragment thereof not containing a lysine or cysteine residue (in the protein form) and to chemically couple (or add) an amino acid TAG containing lysine and/or cysteine among other amino acids to said spider silk protein.

Furthermore, the above method also comprises the option to design modified spider silk protein encoding sequences by substitution and to subsequently add a nucleic acid sequence containing nucleic acids encoding lysine and/or cysteine to said sequence.

The kind and origin of the spider silk protein used in step a) is not restricted as long as it fulfils the requirement not to contain lysine or cysteine residues. It does not play any role whether they are naturally derived or artificial sequences.

The term "fragment" as used herein is directed to parts of spider silk proteins (whether artificial/synthetic or naturally derived) having a length of about 5-50 amino acid residues, preferably 10-40, for example between 20 and 30 amino acid residues.

In a preferred embodiment, the method further comprises coupling other substances to said lysine and/or cysteine molecules in the modified spider silk protein. As mentioned above, this will lead to a controlled and targeted coupling pattern of said substances to the spider silk proteins.

The one or more amino acids replaced in step b) are preferably selected from the group consisting of glycine, alanine, serine, glutamate, aspartate and threonine. They usually do not unduly alter the resulting modified spider silk protein as regards assembly behaviour etc.

The substance to be coupled to said lysine and/or cysteine residues contained in the modified spider silk protein is preferably selected from the group consisting of polypeptides, polysaccharides, marker molecules, quantum dots, metals, nucleic acids, lipids and low molecular drugs.

For example, nanogold particles can be coupled to cysteine residues via a chemical linker. In this case, a covalent coupling is achieved via a maleimido or iodoacetamide group of the linker to the thiol group of cysteine. Basically, all substances can be coupled which are capable to covalently bind to the amino group of lysine or the thiol group of cysteine.

Preferably, the low molecular drugs are selected from drugs containing a carboxyl, carbonyl, imido or thiol group. A not restricted selection of drugs is diclofenac, indomethacine, tolmetine, ibuprofene, flurbiprofene, fenoprofene, naproxene, ketoprofene, penicillines, or cephalosporines.

Preferably, the spider silk protein provided in a) is based on a dragline and/or flagelliform protein. The spider silk sequences could for example be derived from orb-web spiders (Araneidae and Araneoids).

More preferably the spider silk proteins are derived from one or more of the following spiders: Arachnura higginsi, Araneus circulissparsus, Araneus diadematus, Argiope picta, Banded Garden Spider (Argiope trifasciata), Batik Golden Web Spider (Nephila antipodiana), Beccari's Tent Spider (Cyrtophora beccarii), Bird-dropping Spider (Celaenia excavata), Black-and-White Spiny Spider (Gasteracantha kuhlii), Black-and-yellow Garden Spider (Argiope aurantia), Bolas Spider (Ordgarius furcatus), Bolas Spiders - Magnificent Spider (Ordgarius magnificus), Brown Sailor Spider (Neoscona nautica), Brown-Legged Spider (Neoscona rufofemorata), Capped Black-Headed Spider (Zygiella calyptrata), Common Garden Spider (Parawixia dehaani), Common Orb Weaver (Neoscona oxancensis), Crab-like Spiny Orb Weaver (Gasteracantha cancriformis (elipsoides)), Curved Spiny Spider (Gasteracantha arcuata), Cyrtophora moluccensis, Cyrtophora pamasia, Dolophones conifera, Dolophones turrigera, Doria's Spiny Spider (Gasteracantha doriae), Double-Spotted Spiny Spider (Gasteracantha mammosa), Double-Tailed Tent Spider (Cyrtophora exanthematica), Aculeperia ceropegia, Eriophora pustulosa, Flat Anepsion (Anepsion depressium), Four-spined Jewel Spider (Gasteracantha quadrispinosa), Garden Orb Web Spider (Eriophora transmarina), Giant Lichen Orbweaver (Araneus bicentenarius), Golden Web Spider (Nephila maculata), Hasselt's Spiny Spider (Gasteracantha hasseltii), Tegenaria atrica, Heurodes turrita, Island Cyclosa Spider (Cyclosa insulana), Jewel or Spiny Spider (Astracantha minax), Kidney Garden Spider (Araneus mitificus), Laglaise's Garden Spider (Eriovixia laglaisei), Long-Bellied Cyclosa Spider (Cyclosa bifida), Malabar Spider (Nephilengys malabarensis), Multi-Coloured St Andrew's Cross Spider (Argiope versicolor), Ornamental Tree-Trunk Spider (Herennia ornatissima), Oval St. Andrew's Cross Spider (Argiope aemula), Red Tent Spider (Cyrtophora unicolor), Russian Tent Spider (Cyrtophora hirta), Saint Andrew's Cross Spider (Argiope keyserlingi), Scarlet Acusilas (Acusilas coccineus), Silver Argiope (Argiope argentata), Spinybacked Orbweaver (Gasteracantha cancriformis), Spotted Orbweaver (Neoscona domiciliorum), St. Andrews Cross (Argiope aetheria), St. Andrew's Cross Spider (Argiope Keyserlingi), Tree-Stump Spider (Poltys illepidus), Triangular Spider (Arkys clavatus), Triangular Spider (Arkys lancearius), Two-spined Spider (Poecilopachys australasia), Nephila species, e.g. Nephila clavipes, Nephila senegalensis, Nephila madagascariensis and many more (for further spider species, see also below).

Most preferred, the dragline proteins are derived from Araneus diadematus and the flagelliform proteins are derived from Nephila clavipes.

Preferred dragline sequences are ADF-3 and ADF-4. The term ADF-3/-4 is used in the context of MaSp proteins produced by Araneus diadematus (Araneus diadematus fibroin-3/-4). Both proteins, ADF-3 and -4 belong to the class of MaSp II proteins (major ampullate spidroin II).

According to a further embodiment the fragment is a module, wherein the module comprises one or more polyalanine containing consensus sequences. This polyalanine containing consensus sequence is preferably derived from ADF-3 and has the amino acid sequence of SEQ ID NO: 1 (module A) or a variant thereof.

According to a still further embodiment the fragment is a module derived from ADF-3 and comprises the amino acid sequence of SEQ ID NO: 2 (module Q) or a variant thereof. Also combined sequences of the above (and all other modules mentioned herein) are contemplated. For example, a fragment is provided in step a) which comprises one or more of (AQ) and/or (QAQ). Preferably the spider silk protein in this case comprises (AQ)₁₂, (AQ)₂₄, (QAQ)₈ or (QAQ)₁₆.

Thus, the specific modules can also be combined with each other, i.e. modules (repeat units) combining A and Q, Q and C etc. are also encompassed.

Although the number of the modules to be introduced in the spider silk protein is not restricted, it is preferred to employ a number of modules of the synthetic repetitive sequence for each recombinant protein which number is preferably ranging from 5-50 modules, more preferably 10-40 and most preferably between 15-35 modules.

Another preferred module is derived from ADF-4 and comprises the amino acid sequence of SEQ ID NO: 3 (module C) or a variant thereof. Combined sequences can provided in a) may preferably comprise C₁₆ or C₃₂.

Preferred modules derived from a flagelliform protein are module K (SEQ ID NO: 4), module sp (SEQ ID NO: 5), module X (SEQ ID NO: 6), and module Y (SEQ ID NO: 7).

Preferred combinations comprise Y₈, Y₁₆, X₈, X₁₆, K₈, K₁₆ or Y₆X₂sp₁K₂Y₂.

The following new modules were generated by way of the above method and are in particular preferred embodiments:

### Modules for dragline spider silk

**Modul A^{c}:** GPYGPGASAA AAAAGGYGPG CGQQ (SEQ ID NO: 8)
   ggt ccg tac ggc cca ggt gct agc gcc gca gcg gca gcg gct ggt ggc tac ggt ccg ggc tgc ggc cag cag
**Modul A^{K}**GPYGPGASAA AAAAGGYGPG KGQQ (SEQ ID NO: 9)
   ggt ccg tac ggc cca ggt gct agc gcc gca gcg gca gcg gct ggt ggc tac ggt ccg ggc aaa ggc cag cag
**Modul C^{c}:** GSSAAAAAAA ASGPGGYGPE NQGPCGPGGY GPGGP (SEQ ID NO: 10)
**Modul C^{K1}:** GSSAAAAAAA ASGPGGYGPE NQGPKGPGGY GPGGP (SEQ ID NO: 11)
**Modul C^{K2}:** GSSAAAAAAA ASGPGGYGPK NQGPSGPGGY GPGGP (SEQ ID NO: 12)
**Modul C^{KC}:** GSSAAAAAAA ASGPGGYGPK NQGPCGPGGY GPGGP (SEQ ID NO: 13)

### Modules for flagelliform spider silk

**Modul sp^{c}:** GGTTIIEDLD ITIDGADGPI TICEELTI (SEQ ID NO: 14)
**Modul sp^{K}:** GGTTIIEDLD ITIDGADGPI TIIVEELT1 (SEQ ID NO: 15)
**Modul X^{c}:** GGAGGAGGAG GCGGAGGS (SEQ ID NO: 16)
   ggt ggc gct ggt ggc gcc ggt ggc gca ggt ggc tgc ggc ggt gcg ggc ggt tcc
**Modul X^{K}:** GGAGGAGGAG GKGGAGGS (SEQ ID NO: 17)
   ggt ggc gct ggt ggc gcc ggt ggc gca ggt ggc aaa ggc ggt gcg ggc ggt tcc
**Modul Y^{C}:** GPGGAGPGGY GPGGSGPGGY GPGGCGPGGY (SEQ ID NO: 18)
**Modul Y^{K}:** GPGGAGPGGY GPGGSGPGGY GPGGKGPGGY (SEQ ID NO: 19)

These modules can be combined purposely with other modules/spider silk proteins in order to achieve a specific modification. As an example for the possibilities of combinations, construct C₁₆ is used. In this respect, possible constructs by use of module C^{c} could be the following:
C₁₆C^{C}, C^{C}C₁₆, C₈C^{C}C₈, C₁₆^{C}, C₈C^{C}₈, C^{C}₈C₈, C₄C^{C}₈ C_{4,} C^{C}₄C₈C^{C}₄, etc.

In these constructs a controlled and targeted coupling can be achieved via the thiol group of cysteine as well as via the amino groups of lysine. For example, by a combination of C^{c} with module C^{K1}, the possibility is existing to couple appropriate substances to the thiol groups of cysteine and to the amino groups of the lysine side chains.

Preferred constructs could be designed as follows: for example, both amino acids are introduced in one single module (module C^{KC}) which opens up further possibilities (an enormous number of construct variants is occurring).

Since it cannot be ruled out that in single cases the amino acid exchange can lead to an alteration of the assembly characteristics or to modified characteristics of the construct, as a further preferred alternative, specific TAGs may be used. These tags (for example TAG's as disclosed in SEQ ID NO: 20-28, below) contain cysteine or lysine as mentioned before. The sequence of the TAG is so selected that an interaction with the rest of the protein and an influence of the assembling behaviour can be precluded to the greatest possible extent.

Thus, according to a preferred embodiment, the modified spider silk protein recovered in step d) comprises one or more of the modules of SEQ ID NO: 8-19.

The following TAG's were developed for preferred use in the spider silk constructs:

### amino terminal TAGs

**NH^{CYSI}:** GCGGGGGGSG GGG (SEQ ID NO: 20)
   ggt tgc ggc ggt ggc ggt ggc ggt tct ggt ggc ggt ggc
**NH^{CYS2}:** GCGGGGGG (SEQ ID NO: 21)
   ggt tgc ggt ggc ggt ggc ggt ggc
**NH^{CYS3} :** GCGG S GGGG S GGGG (SEQ ID NO: 22)
   ggt tgc ggt ggc tct ggt ggt ggc ggg tcc gga ggc ggt ggc
**NH^{LYS1}:** GKGGGGGGSG GGG (SEQ ID NO: 23)
   ggt aaa ggc ggt ggc ggt ggc ggt tct ggt ggc ggt ggc
NH^{LYS2}: GKGGGGGG (SEQ ID NO: 24)
   ggt aaa ggt ggc ggt ggc ggt ggc

### carboxylic terminal TAGs

**CH^{CYS1}:** GGGGSGGGGS GGCG (SEQ ID NO: 25)
   ggc ggt ggc ggt tct ggc ggt ggc ggt tct ggc ggt tgc ggc
**CH^{CYS2}:** GGGGSGGCG (SEQ ID NO: 26)
   ggc ggt ggc ggt tct ggc ggt tgc ggc
**CH^{LYS1}:** GGGGSGGGGS GGKG (SEQ ID NO: 27)
   ggc ggt ggc ggt tct ggc ggt ggc ggt tct ggc ggt aaa ggc
**CH^{LYS2}:** GGGGSGGKG (SEQ ID NO: 28)
   ggc ggt ggc ggt tct ggc ggt aaa ggc

As also mentioned above, as an example, the following different variants can be used:
NH^{CYS1}C₁₆, C₁₆CH^{CYS1}, NH^{CYS1}C₁₆CH^{LYS1}, NH^{LYS1}C₁₆CH^{CYS1}, etc.

Replacing nucleic acids which are encoding one or more amino acids in spider silk proteins by lysine or cysteine can lead to changes in the characteristics of the resulting modified spider silk protein. In order to avoid unwanted modifications, the skilled artisan knows how to chose the specific position of the substitution reaction in order to avoid those unwanted alterations or in order to introduce further wanted characteristics into the spider silk protein sequence. Therefore, it is in particular preferred to use non hydrophobic amino acids which are neutral, e.g. are not carrying any charges in the amino acid side chains. The amino acids to be replaced into the original spider silk protein sequence additionally should have a comparable size in order to avoid a steric hindrance due to the newly introduced amino acids. Therefore, it is in particular preferred to use serine, alanine, glycine, glutamate, aspartate or threonine to be substituted by lysine or cysteine.

Thus, to the modified spider silk protein recovered in step d) or to the spider silk protein provided in step a) a nucleic acid encoding an amino terminal TAG according to SEQ ID NO: 20-24 and/or a carboxyl terminal TAG of SEQ ID NO: 25-28 may be added.

As explained above, the amino acid sequences disclosed herein are not restricted to the exact sequences provided in the SEQ ID Nos. The amino acid sequences indicated herein also comprise variants. Thus, the amino acid sequences of the proteins of the present invention also encompass all sequences differing from the herein disclosed sequences by amino acid insertions, deletions, and substitutions.

Preferably; amino acid "substitutions" are the result of replacing one amino acid with another amino acid having similar structural and/or chemical properties, i.e., conservative amino acid replacements. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

"Insertions" or "deletions" are typically in the range of about I to 5 amino acids, preferably about 1, 2 or 3 amino acids. Amino acid additions typically are not more than 100, preferably not more than 80, more preferably not more than 50, most preferred not more than 20 amino acids, which are added on and/or inserted into the proteins of the present invention. It is noted that only those additions are contemplated in this invention, which do not negatively affect the desired characteristics of the proteins disclosed herein.

The variation allowed may be experimentally determined by systematically making insertions, deletions, or substitutions of amino acids in a protein using recombinant DNA techniques and assaying the resulting recombinant variants for activity. This does not require more than routine experiments for the skilled artisan.

It is noted that the present method additionally may comprise the step of spinning said proteins prepared in step d) into filaments, nanofibers and threads by a suitable method.

For this purpose, spinning methods may be used, which are per se known in the art. For example, a dope solution of spider silk protein is extruded through a spinneret to form a biofilament. The resulting biofilament can be drawn or stretched. Whenever both crystalline and amorphous arrangements of molecules exist in biofilaments, drawing or stretching will apply shear stress sufficient to orient the molecules to make them more parallel to the walls of the filament and increase the tensile strength and toughness of the biofilament.

Preferably, the dope solution is at least 1%, 5%, 10%, 15% weight/volume (w/v) modified silk protein. More preferably, the dope solution is as much as 20%, 25%, 30%, 35%, 40%, 45%, or 50% w/v silk protein. In preferred embodiments, the dope solution contains substantially pure modified spider silk protein. In preferred embodiments, the dope has a pH of approximately 6.9.

By "dope solution" is meant any liquid mixture that contains silk protein and is amenable to extrusion for the formation of a biofilament or film casting. Dope solutions may also contain, in addition to protein monomers, higher order aggregates including, for example, dimers, trimers, and tetramers. Normally, dope solutions are aqueous solutions of pH 4.0-12.0 and having less than 40% organics or chaotropic agents (w/v). Preferably, the dope solutions do not contain any organic solvents or chaotropic agents, yet may include additives to enhance preservation, stability, or workability of the solution.

By "filament" is meant a fibril of indefinite length, ranging from nanoscale and microscopic length to lengths of a mile or greater. Silk is a natural filament, while nylon and polyester as an example are synthetic filaments.

Further information regarding how to spin spider silk protein fibrils may be found in WO03060099 (Karatzas et al.), Published July 24, 2003.

Furthermore, the modified spider silk proteins of the present invention may be provided as films or the like, i.e. as a spider silk protein product, for which a spinning step is not required.

As already mentioned, the modified spider silk protein according to the first aspect of the invention can be obtained by the above method.

A preferred modified spider silk protein comprises one or more of the modules of SEQ ID NO: 8 to 12 or SEO ID NO: 14 to 28.

According to a second aspect, a nucleic acid sequence is provided coding for a modified spider silk protein according to the first aspect of the invention, preferably for a modified spider protein as mentioned above.

The term "nucleic acid sequence" refers to a heteropolymer of nucleotides or the sequence of these nucleotides. The terms "nucleic acid" and "polynucleotide" are used interchangeably herein to refer to a heteropolymer of nucleotides.

Stringency of hybridization, as used herein, refers to conditions under which polynucleotide duplexes are stable. As known to those of skill in the art, the stability of duplex is a function of sodium ion concentration and temperature (see, for example, Sambrook et al., Molecular Cloning: A Laboratory Manual 2nd Ed. (Cold Spring Harbor Laboratory, (1989)). Stringency levels used to hybridize can be readily varied by those of skill in the art.

As used herein, the phrase "moderately stringent conditions" refers to conditions that permit DNA to bind a complementary nucleic acid that has about 60% identity, preferably about 75% identity, more preferably about 85% identity to the DNA; with greater than about 90% identity to said DNA being especially preferred. Preferably, moderately stringent conditions are conditions equivalent to hybridization in 50% formamide, 5 x Denhart's solution, 5 x SSPE, 0.2% SDS at 42°C, followed by washing in 0.2 x SSPE, 0.2% SDS, at 65°C.

A third aspect is directed to an expression vector, which comprises the above defined nucleic acid sequence and one or more regulatory sequences. This expression vector preferably comprises one or more regulatory sequences. The term "expression vector" generally refers to a plasmid or phage or virus or vector, for expressing a polypeptide/protein from a DNA (RNA) sequence. An expression vector can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an amino-terminal methionine residue. This residue may or may not be subsequently cleaved from the expressed recombinant protein to provide a final product.

The vector preferably is a plasmid or a viral vector, preferably a baculovirus system or a vaccinia virus vector system. Further viral vector systems may also be used in this invention. From case to case, a modification of the vector may be needed. Examples for further viral vectors are adenoviruses and all negative-strand RNA-viruses, e.g. rabies, measles, RSV, etc.

Ready-to-use genetic constructs can be cloned in different commercially available expression vectors as for example pET (Novagen, Madison, Wisconsin, USA) or the pQE-systems (Qiagen GmbH, Hilden, Germany). By the specific choice of the vectors or of the restriction enzymes which are used for cloning, different protein TAGs can be attached to the protein (for example T7-tag (Novagen, Madison, Wisconsin, USA) or the 6xhistidin-tag. Furthermore, one can choose between different promotors (for example T7 or T5).

Preferably, the vector comprises the above nucleic acid sequence coding for a modified spider silk protein and preferably is derived from the cloning vector of SEQ ID NO: 29 (cloning vector pAZL) or a variant thereof.

A fourth aspect of the invention is related to a host, which has been transformed with the above vector. The host may be a prokaryotic cell, preferably *E.coli* or *Bacillus subtilis.* The expression of the synthetic gene can be performed for example in *E*. *coli K12* or *E*. *coli B* cells. The yield of the expression can be about 1 g of purified protein per litre of bacterial culture.

The host may also be a eukaryotic cell, for example a mammalian cell, plant cell, yeast cell or an insect cell. Preferably, it can be a CHO, COS, HeLa, 293T, HEH or BHK cell, a yeast cell (for example *Saccharomyces cerevisiae, Schizosaccharomyces pombe, Pichia pastoris, Candida albicans, Hansenula polymorpha*)*,* an insect cell selected from *Lepidoptera* insect cells, preferably from *Spodoptera frugiperda* and from *Trichoplusia ni,* more preferably a Sf9, Sf21 or high five cell, or a plant cell, preferably derived from tobacco, potato, corn, pea and tomato.

One advantage of an insect cell expression system, for example regarding bacterial systems, resides in the fact that the proteins produced are glycosylated, thereby being a target for degradation by micro organisms. This characteristic may be of importance, for example, in the field of medicine, whenever the silk proteins are intended for an *in vivo* use, in which biological degradation is desired. This characteristic may in particular find application in suture materials and wound closure and coverage systems.

In a fifth aspect, the present invention is directed to fibrils/threads or filaments made from the above modified spider silk protein.

The proteins, threads, filaments, films, foams, spheres, nanofibrils, hydrogels and the like as defined herein may be used in the field of biotechnology and/or medicine, preferably for the manufacture of wound closure or coverage systems, suture materials for use in neurosurgery or ophthalmic surgery. Furthermore, the proteins/threads may preferably be used for the manufacture of replacement materials, preferably artificial cartilage or tendon materials.

Additionally, the threads/fibrils of the invention can be used in the manufacture of medical devices such as medical adhesive strips, skin grafts, replacement ligaments, and surgical mesh; and in a wide range of industrial and commercial products, such as clothing fabric, bullet-proof vest lining, container fabric, bag or purse straps, cable, rope, adhesive binding material, non-adhesive binding material, strapping material, automotive covers and parts, aircraft construction material, weatherproofing material, flexible partition material, sports equipment; and, in fact, in nearly any use of fibrils or fabric for which high tensile strength and elasticity are desired characteristics. Adaptability and use of the stable fibril product in other forms, such as a dry spray coating, bead-like particles, or use in a mixture with other compositions is also contemplated by the present invention.

As mentioned above, pharmaceutical substances may be coupled to the modified spider silk proteins of the invention via their cysteine/lysine residues. Especially those coupled proteins can be used for the above purposes. For example, an envisioned application of such a coupled protein is the manufacture of a suture material or wound coverage system having antibiotics or anti-inflammatory drugs attached to the proteins/threads, from which they were made.

It is explicitly noted that the most preferred applications of the modified spider silk proteins of the present invention are in the manufacture and processing of clothing fabric (textiles) and leather, automotive covers and parts, aircraft construction materials as well as in the manufacture and processing of paper.

The modified spider silk proteins of the present invention may be added to cellulose and keratin and collagen products and thus, the present invention is also directed to a paper or a skin care and hair care product, comprising cellulose and/or keratin and/or collagen and the spider silk proteins of the present invention. Papers and skin care and hair care products, in which the proteins of the present invention are incorporated are showing improved characteristics, in particular improved tensile strength or tear strength.

Furthermore, the modified spider silk proteins of the invention may be used as a coating for textile and leather products, thereby conferring stability and durability to the coated product. The silk proteins in particular show applicability for coating leather products, since in this case, tanning and its negative effects for environment can be avoided or at least reduced.

They can also be used in food packaging or electronic devices, for example in batteries. Experiments conducted with films made out of the modified spider silk proteins showed their resistance and stability towards acid after immersion in battery acid.

In a sixth aspect, the invention provides a pharmaceutical or cosmetical composition containing a modified spider silk protein as defined hereinabove and a pharmaceutically acceptable carrier.

The invention is further illustrated by the accompanying figures, wherein:
- Fig. 1: is showing the schematic structure of a cloning cassette used in the present invention;
- Fig. 2: illustrates an example of the multimerization of the DNA modules of the present invention.

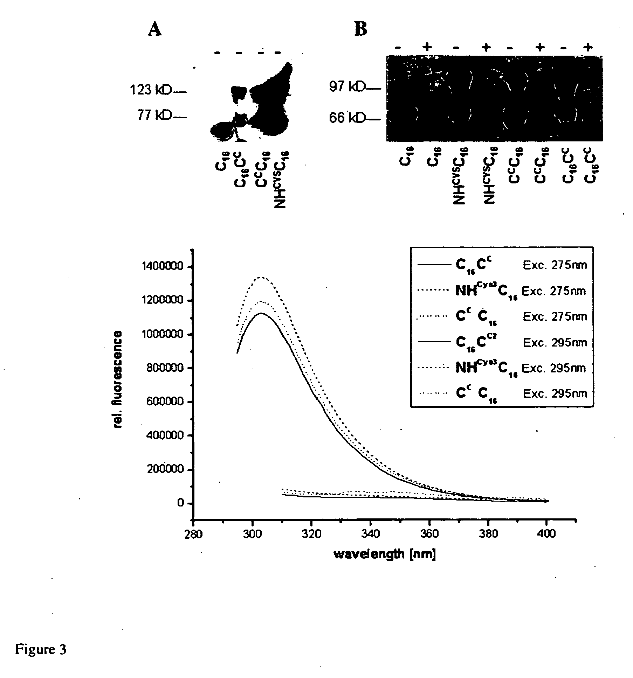

Fig. 3 reveals the analysis of modified spider silk proteins. The samples were analysed with (+) or without (-) reducing agent (2-mercaptoethanol). (**A**) T7-tags of recombinant silk proteins were detected after western blotting with an anti-T7-tag antibody. (**B**) Proteins were subjected to SDS-PAGE followed by silver staining. (**C**) Fluorescence emission spectra of purified C₁₆C^{C} (straight line), NH^{CYS3}C₁₆ (long dashes) and C^{C}C₁₆ (dotted line) are shown with excitation wavelengths of 275 nm or 295 nm, respectively. Excitation at 295 nm reveals the absence of tryptophane fluorescence. Therefore, the protein samples do not exhibit any detectable contamination of bacterial proteins.

Fig. 4 shows the secondary structure analysis of modified spider silk proteins. CD spectra of C₁₆ (straight line), C₁₆C^{C} (long dashes), NH^{CYS3}C₁₆ (dots and dashes) and C^{C}C₁₆ (dotted line) were recorded at 20°C.

Fig. 5 depicts the aggregation of modified synthetic spider silk proteins. Aggregation of proteins was determined after incubation for 2 hours in buffers with (**A**) varying pH or (**B**) varying potassium phosphate concentrations (see table 2). The obtained curve for C₁₆ is represented by squares, the one for NH^{CYS3}C₁₆ by circles, for C^{C2}C₁₆ by triangles and the one for C₁₆C^{C2} by reverse triangles.

Fig. 6 reveals assembly forms of modified spider silk proteins. (**A**) Spheres formed by C₁₆ and modified proteins visualized by scanning electron microscopy (SEM). (**B**) Nanofibrils visualized by atomic force microscopy. (**C**) Film cast from a 1% w/v C^{C2}C₁₆ solution in HFIP.

Fig. 7 shows CD spectra of protein films made from modified spider silk proteins. (**A**) Protein solution in HFIP was analysed before film casting. (**B**) Films were cast from HFIP directly on a plain quartz glass and analysed by CD spectroscopy. (**C**) CD analysis of films cast from HFIP processed with methanol (exemplarily shown for NH^{CYS3}C₁₆). (**D**) Analysis of films cast from formic acid directly on a plain quartz glass. Due to inaccuracies in defining the thickness of the films, Θ_{MRW} could not be determined.

Fig. 8 depicts coupling of Rhodamine Red™ C₂ maleimide to NH^{CYS}C₁₆. The coupled protein was analysed by SDS-PAGE. (**A**) Proteins were visualised by silver staining. (**B**) Rhodamine was visualised by fluorescent imaging.

### EXAMPLES

### EXPERIMENTAL PROCEDURES

*Materials.* Chemicals were obtained from Merck KGaA (Darmstadt, Germany) if not otherwise stated. Manipulation and modification of DNA was performed as described previously (1). Restriction enzymes were obtained from New England Biolabs (Beverly, MA, USA) and ligase from Promega Biosciences Inc. (San Luis Obispo, CA, USA). DNA purification was performed using kits from Promega Biosciences Inc. (San Luis Obispo, CA, USA). Synthetic oligonucleotides were obtained from MWG Biotech AG (Ebersberg, Germany). All cloning steps were performed in the *E*. *coli* strain DH10B from Novagen (Madison, WI, USA).

### Cloning of modified silk modules and TAGs into the pAZL vector.

Module C^{c} (SEQ ID NO: 10), was created via PCR mutagenesis using Module C (SEQ ID NO: 3) as template nucleotide sequence and primers pAZL-fwd (CACTGAGCGTCAGA CCCCGTAGAAAAGA) (SEQ ID NO: 30) and pAZLmut-rev (CTCTTAAGCTT TCATTAGCCTGGACCACCTGGACCGTAGCCGCCCGGGCCGCAAGGACCCTGG) (SEQ ID NO: 31). In order to obtain an optimized primer some codons of the original Modul C were mutated (CCA (Pro24) to CCT (Pro), GGT (Gly28) to GGC (Gly), CCT (Pro32) to CCA (Pro), GGC (Gly33) to GGT and CCG (Pro35) to CCA (Pro)). The PCR-product and pAZL vector (SEQ ID NO: 29) were ligated after digestion with *Alw*NI and *Hind*III. Module NH^{CYS3} (GCGG S GGGG S GGGG, ggt tgc ggt ggc tct ggt ggt ggc ggg tcc gga ggc ggt ggc) (SEQ ID NO: 22) was created by annealing two synthetic oligonucleotides N 1 (GATCCATGGGTTGCGGTGGCTCTGGTGGTGGCGGGTCCG GAGGCGGTGGCTAATGAA) (SEQ ID NO: 32) and N2 (AGCTTTCATTAGCCACCGCCTCC GGACCCGCCACCACCAGAGCCACCGCAACGCATG) (SEQ ID NO: 33). Annealing was accomplished by decreasing the temperature of a 50 pmol/µl (each) oligonucleotide solution from 95°C to 20°C with an increment of 0.1°C/s. Mismatched double strands were denatured at 70°C followed by another temperature decrease to 20°C. After repeating the 20°C-70°C-20°C cycle ten times, ten additional cycles were performed with a denaturing temperature of 65°C. The resulting cloning cassette was ligated with vector pAZL (SEQ ID NO: 29) digested with *Bam*HI and *Hind*III.

*Construction of modifed synthetic spider silk genes.* Connecting of two gene fragments e.g. single modules or module multimers represented the basic step of the cloning strategy. For this purpose the pAZL vector, containing the designated 5'-terminal gene fragment was digested with *BsaI* and *BsgI*, while the vector comprising the 3'-terminal gene fragment was digested with *BseRI* and *BsaI* respectively (Fig.1). Ligation of the appropriate plasmid fragments yielded the connecting of the two gene fragments and led to the reconstitution of the pAZL vector's ampicillin resistance gene (*Ap'*) which facilitated identification of correct constructs.

For gene construction, modified modules C^{C} (SEQ ID NO: 10) or NH^{CYS3} (SEQ ID NO: 22) were connected to repeat units like C₁₆. Afterwards, they were excised from the pAZL vector with *BamHI* and *HindIII* and ligated with the bacterial expression vector pET21 a (Novagen) likewise digested, providing a T7-tag (MASMTGGQQMGR) (SEQ ID NO: 34) coding sequence (2). The fidelity of all constructs was confirmed by DNA sequencing.

*Gene expression.* All silk genes were expressed in the *E. coli* strain BLR [DE3] (Novagen). Cells were grown at 37°C in LB medium to an OD₆₀₀ = 0.6-0.7. After induction with 1 mM IPTG (Isopropyl-β-D-thiogalactosid), cells were shifted to 25°C in the case of NH^{CYS3}C₁₆ and C₁₆C^{C} and to 30°C in the case of C^{C}C₁₆, respectively. Cells expressing NH^{CYS3}C₁₆ were harvested after 3-4 hours of induction while cells expressing C^{C}C₁₆ were harvested after 4 hours and cells expressing C₁₆C^{C} after 5 hours.

*Protein purification.* Cells were resuspended with 5 ml/g buffer containing 20 mM *N*-(2-hydroxyethyl)piperazine-N'-(2-ethanesulfonic acid) (HEPES) pH 7.5, 100 mM NaCl, 0.2 mg/ml lysozyme (Sigma-Aldrich, St. Louis, MO, USA) and incubated at 4°C for 30min. Cells were disrupted by high pressure using a French Press (Basic Z Model, APV Deutschland GmbH, Lübeck, Germany). Genomic DNA was digested by incubating cell lysates with 0.1 mg/ml DNase I (Roche, Mannheim, Germany) and 3 mM MgCl₂ at room temperature for 30 min. Insoluble cell fragments were sedimented at 50,000×g and 4°C for 30 min. Soluble *E. coli* proteins of lysates were precipitated by heat denaturation at 80°C for 20 min. Precipitated proteins were removed by sedimentation at 50,000×g for 30 min. Silk proteins, which remained soluble during heat denaturation, were precipitated with 20% ammonium sulphate (800 mM) at room temperature and harvested by centrifugation at 10,000×g for 10 min. Pellets were rinsed with 8 M urea and dissolved in 6 M guanidinium thiocyanate (GdmSCN). All proteins were dialyzed against 10 mM NH₄HCO₃. Precipitates formed during dialysis were removed by sedimentation at 50,000×g for 30 min and the remaining soluble silk proteins were lyophilized. Prior to analysis lyophilized protein was dissolved in 6 M GdmSCN followed by dialysis against appropriate buffers. Aggregates were removed by sedimentation at 125,000×g for 30 min. Protein concentrations were determined photometrically in a 1 cm path length cuvette at 276 nm using calculated extinction coefficients (Table 1) (3). Identity of proteins was confirmed by sodium dodecylsulfate - polyacrylamide gel electrophoresis (SDS-PAGE; 10% Tris-Glycine gels) followed by blotting onto polyvinylidene fluoride (PVDF) membranes (Millipore, Billerica, MA, USA) and detection using a mouse anti-T7 monoclonal antibody (Novagen, 1:10,000) as primary and anti-mouse IgG peroxidase conjugate (Sigma-Aldrich, 1:5,000) as secondary antibody. Peroxidase activity was visualized using the ECL^{plus} western blot detection kit from Amersham Biosciences (Piscataway, NJ, USA).

**Table 1: Extinction coefficients of synthetic spider silks (calculated according to Gill & Hippel (3)).**

| | C¹⁶ | NH^{CYS3}C₁₆ | C^{C}C₁₆/C₁₆C^{C} |
|---|---|---|---|
| extinction coefficient (276 nm) [M⁻¹cm⁻¹] | 46 400 | 46 400 | 49 300 |

*Fluorescence.* Fluorescence spectra were recorded on a FluoroMax 3 Spectrofluorometer (Jobin Yvon Inc, Edison, NJ, USA). Spectra were taken in 10 mM NH₄HCO₃ or 10 mM Tris(hydroxymethyl)aminomethane (Tris) / HCl (pH 8.0) at 25 °C. Integration time was 1 s, step size was 0.5 nm and band widths were 5 nm (excitation) and 5 nm (emission), respectively.

*Secondary structure analysis.* Far-UV circular dichroism (CD) spectra were obtained using a Jasco 715 spectropolarimeter equipped with a temperature control unit (Jasco International Co. Ltd., Tokyo, Japan). Spectra were taken at a protein concentration of 150 µg/ml in 10 mM Tris/HCl (pH 8.0) in a 0.1 cm path length quartz cuvette at 20°C. Scan speed was 20 nm/min, step size was 0.2 nm, integration time was set to 1 s and band width was 1 nm. Four scans were averaged and buffer-corrected.

*Aggregation assay.* To test the solubility of the proteins at different pH-values and different phosphate concentrations, respectively, lyophilized protein was dissolved in 6M GdmSCN and dialysed against 10 mM Tris/HCl (pH 8.0) at 4°C. All samples were incubated at room temperature for 2 hours in different buffers (see below), protein precipitates were removed from the samples by sedimentation at 125,000×g for 30 min and the amount of the remaining soluble protein was determined photometrically. Since the sum of soluble and aggregated protein had to equal the initial amount of soluble protein, the percentage of aggregated protein could be calculated by subtracting the amount of soluble protein from the initially used amount of protein. As a control, proteins were incubated in 10 mM Tris/HCl (pH 8.0).

### Influence of pH-values

Protein solutions were diluted 1:10 with buffers of different pH (Table 2). Final protein concentrations were 0.2 mg/ml in case of NH^{CYS3}C₁₆ and C^{C}C₁₆ and 0.174 mg/ml in case of C₁₆C^{C}.

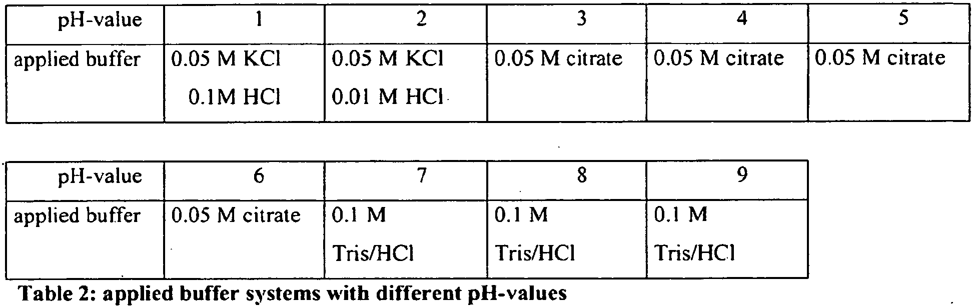

### Influence of phosphate concentration

Protein solutions were diluted 1:5 in KₓHₓPO₄ (pH 8.0). Final protein concentrations were 0.4 mg/ml and phosphate concentrations were 50 mM, 100 mM, 200 mM, 300 mM and 500 mM.

*Coupling of Rhodamine maleimide to thiol groups.* In order to couple a small organic compound to modified spider silk proteins, the fluorescent dye rhodamine was coupled to the protein NH^{CYS3}C₁₆. A stock solution of 1mM Rhodamine Red™ C₂ maleimide (Molecular Probes, Leiden, The Netherlands) in DMSO was added to a protein solution in 10 mM Tris/HCl (pH 7.5) to give a molar excess of 20. The reaction was carried out over night in the dark at 4°C. To inactivate remaining non-coupled fluorescent dye, 100 mM 2-mercaptoethanol was added to the reaction before size exclusion chromatography (PD10 columns, Sephadex G 25, Pharmacia Biotech, Uppsala, Sweden). Fractions were collected, tested for the presence of protein by UV-spectrometry and finally analysed by SDS-PAGE. Visualization of proteins and the fluorophore was performed by silver staining and by fluorescent imaging using a Typhoon 9200 Variable Mode Imager (Molecular Dynamics, Amersham Pharmacia Biotech, Uppsala, Sweden) with an excitation wavelength of 532 nm and an emission wavelength of 580nm, respectively.

### RESULTS

*Design, synthesis and purification of modified synthetic spider silks.* Different modified modules based on the synthetic spider silk Module C (SEQ ID NO: 3), which is derived from the dragline silk protein ADF-4 from the garden spider *Araneus diadematus*, were created. The modifications contain one cysteine in each of the variants, either in a TAG or in the Module C. Therein, serine at position 25 was mutated to cysteine due to similar size and polarity of both amino acids. Additionally, the position was considered not to affect protein characteristics too much, as hydrophobicity predictions for this mutation differ only slightly from Module C. The resulting Module C^{C} (SEQ ID NO: 10) was gained using PCR mutagenesis (see experimental procedures). In a following step, the nucleotide sequence of Module C^{C} was cloned *upstream* or *downstream* the nucleotide sequence coding for C₁₆ yielding proteins C^{C}C₁₆ and C₁₆C^{C} using cloning vector pAZL (SEQ ID NO: 29). Additionally, an oligonucleotide sequence encoding a TAG consisting of glycine, serine and one cysteine (NH^{CYS3}, SEQ ID NO: 22) was cloned at the 5'-terminal nucleotide sequence coding for C₁₆ to yield NH^{CYS3}C₁₆.

After bacterial synthesis, modified silk proteins were purified by a heat step followed by an ammonium sulphate precipitation. The identity of the proteins was confirmed by immunoblotting, using antibodies directed against T7-peptide tag sequences, attached to the amino-terminal end of all silk proteins. As comparison the unmodified protein C₁₆ was applied. Besides full length proteins, traces of proteins with lower molecular weight were observed. In contrast to C₁₆, all modified proteins, containing one cysteine each, revealed an additional protein band at higher molecular weight (Fig. 3A and B "-") which could be removed by adding reducing agents like 2-mercaptoethanol to the samples (Fig. 3B "+"). Therefore, they represent protein dimers connected by disulfide bridges. Protein purity was determined by measuring fluorescence emission. Incident light of 275 nm leads to excitation and fluorescence emission of tyrosines and tryptophanes. Light of 295 nm exclusively excites tryptophanes. Since none of the designed spider silk proteins comprised tryptophanes, fluorescence emission upon excitation with 295 nm would have been indicative for contaminating *E. coli* proteins, which on average contain 1.5% of tryptophane (4). Fluorescence measurement of all modified silk protein preparations revealed emission spectra akin the spectrum of tyrosine, which occurs abundantly in the silk proteins. In contrast, no tryptophane fluorescence could be detected, indicating high purity of the protein preparations (Fig. 3C). Yields of pure proteins ranged from 12 to 30 mg per litre of culture medium.

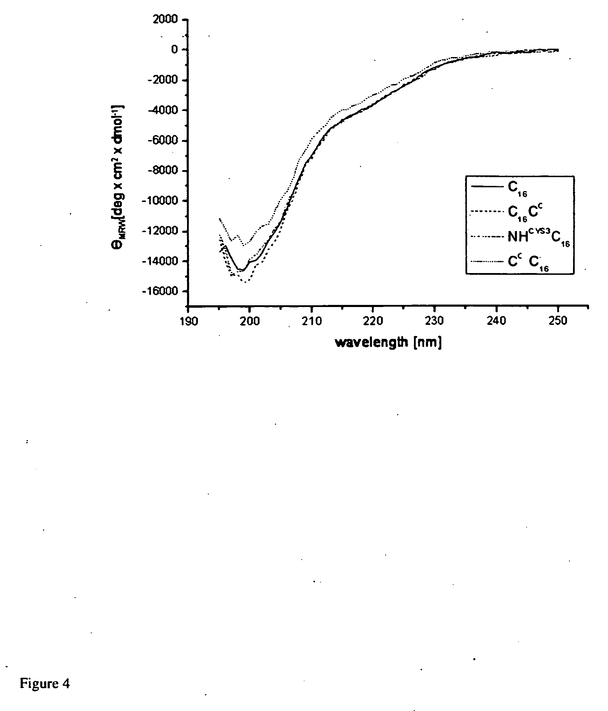

*Modified C₁₆ spider silks show the same secondary structure as non-modified C₁₆*. Secondary structure was investigated by CD spectroscopy. All modified proteins revealed spectra akin to C₁₆, which displays a spectrum typical for intrinsically unstructured proteins (Fig. 4).

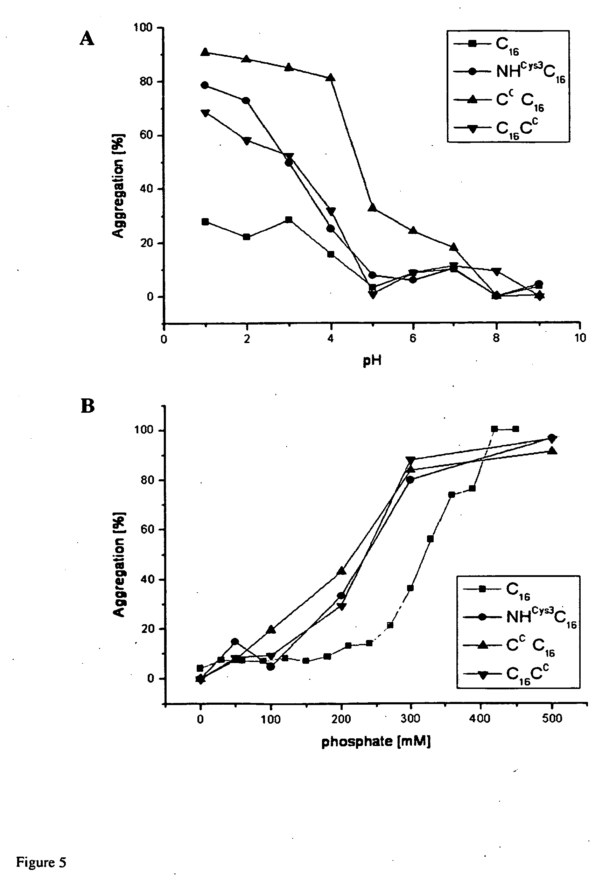

*Modified C₁₆. spider silks are more susceptible towards phosphate and pH than non-modified C₁₆*. pH, ions, such as potassium and phosphate, and mechanical stress are involved in natural silk assembly. We investigated the influence of different potassium phosphate concentrations and varying pH on the aggregation behaviour of modified proteins C^{C}C₁₆, C₁₆C^{C} and NH^{CYS3}C₁₆ in comparison to C₁₆. All modified proteins showed significant aggregation (>10%) at pH-values below 5.0, C^{C}C₁₆ even at values below 7.0 and showed more than 70% aggregation at pH 1. C₁₆, in contrast, only displayed moderate aggregation under these conditions (Fig. 5A). Similarly, potassium phosphate led to aggregation of modified proteins at lower concentrations than those observed for C₁₆ (Fig. 5B). This greater susceptibility for aggregation of the modified proteins cannot be explained by a different charge of the proteins, as their theoretical isoelectric point is identical (NH^{CYS3}C₁₆) to the one of C₁₆ or differs only very slightly (3.45 for C^{C}C₁₆ and 3.48 for C₁₆). Therefore, this effect might be due to the presence of a cysteine and the possibility to form stable dimers.

### Assembly of modified synthetic spider silk proteins

Synthetic spider silk proteins derived from spider silk sequences ADF-3 and ADF-4 can be assembled into morphological distinct forms, like spheres, nanofibrils, foams and films. The following experiments were performed to demonstrate, that modified spider silk proteins show the same features concerning distinct assembly behaviour.

### 1. Spheres

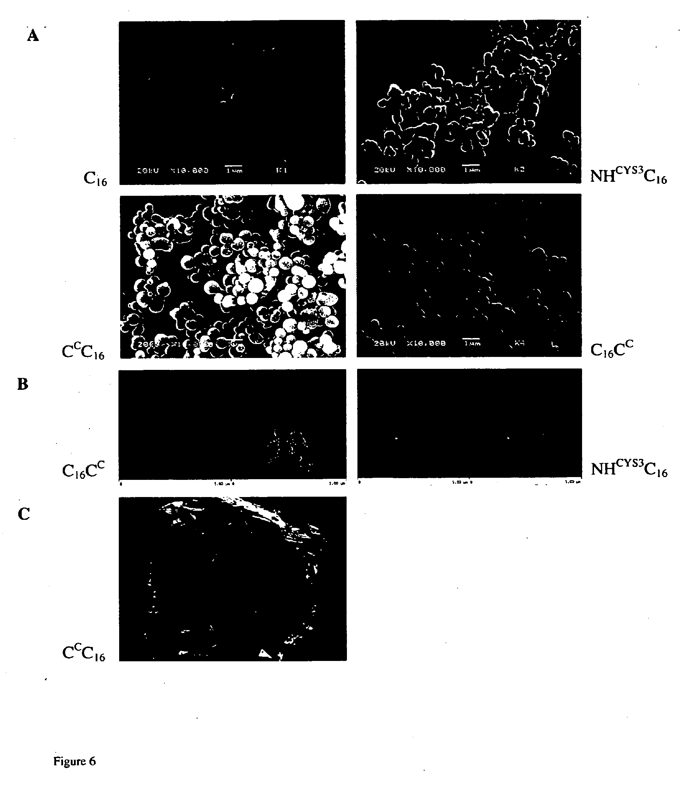

Protein spheres, displaying diameters ranging between 0.3 and 1.5 µm (Fig. 6A), were generated by adding 2.4 M ammonium sulphate to a 1 mg/ml solution of C^{C}C₁₆, C₁₆C^{C}, NH^{CYS3}C₁₆ and C₁₆ in 10 mM Tris-(hydroxymethyl)-aminomethan (Tris) pH 8.0. No significant difference was observed between spheres made from modified proteins or C₁₆.

### 2. Nanofibrils

Nanofibrils were formed by incubating solutions of C₁₆C^{C} and NH^{CYS3}C₁₆ in 10 mM Tris pH 8.0 at 4 °C for several weeks followed by incubation at room temperature for 3 days (Fig. 6B).

### 3. Films

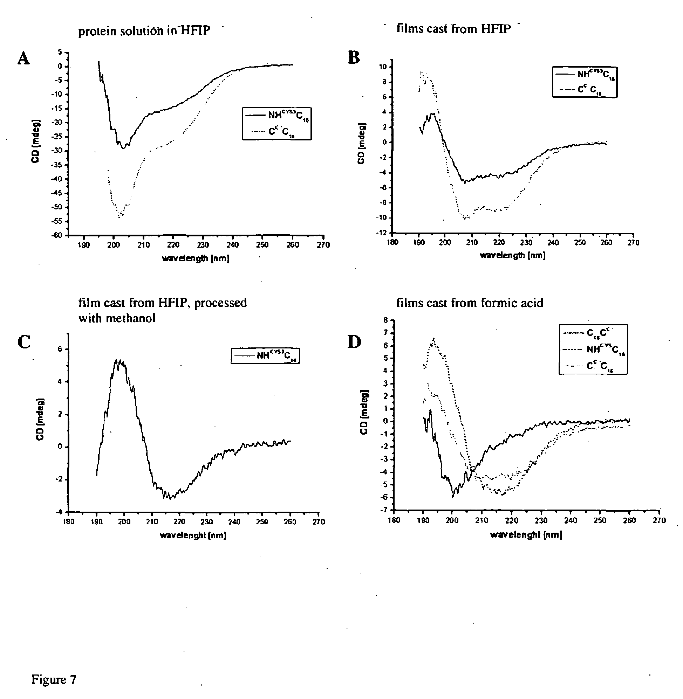

Films made of synthetic spider silk proteins derived from the dragline silk protein ADF-4 from the garden spider *Araneus diadematus* can be cast from hexafluoro-2-propanol (HFIP) or formic acid (5). Lyophilized proteins were directly dissolved in HFIP or formic acid. As observed for C₁₆, HFIP induces an increase in secondary structure of the modified proteins C^{C}C₁₆, C₁₆C^{C} and NH^{CYS3}C₁₆. While CD spectra of these proteins in 10 mM Tris (pH 8.0) showed only a single minimum at a wavelength below 200 nm (Fig. 4), which is indicative of a mainly random coiled protein, CD spectra of protein solutions in HFIP revealed a minimum at 202-203 nm and an additional shoulder at 220 nm, which is indicative of an increased α-helical content (Fig. 7).

From modified spider silk proteins films could be cast out of HFIP as well as from formic acid. Films were cast on a polystyrene surface, where they could be easily peeled off after evaporation of the solvent (Fig. 6C). In order to analyse the secondary structure of the films, a 0.01% w/v protein solution was cast on quartz glass. After the solvent had evaporated, circular dichroism was measured. Films cast from HFIP revealed spectra with two minima at 208 nm and 220 nm, indicative of a high α-helical content (Fig. 7). To render the films cast from HFIP water-insoluble (the as-cast films readily dissolve upon contact with water), they were treated with methanol (5). After this treatment, the spectrum revealed a single minimum at 218 nm which is typical for a β-sheet rich structure (exemplarily shown for NH^{CYS3}C₁₆ Fig. 7). This change in secondary structure upon processing of the films was already described for synthetic spider silk films (5). Interestingly, films cast from formic acid differed in their secondary structure depending on the protein employed. While NH^{CYS3}C₁₆ led to a film displaying a β-sheet rich structure (with a minimum at 218 nm in CD spectrum) like it was described for C₁₆ (5), the spectrum of a film from C^{C}C₁₆ revealed two minima at 208 nm and 220 nm. C₁₆C^{C} protein films revealed CD spectra with a single minimum at 200 nm.

### 4. Labelling of modified synthetic spider silks

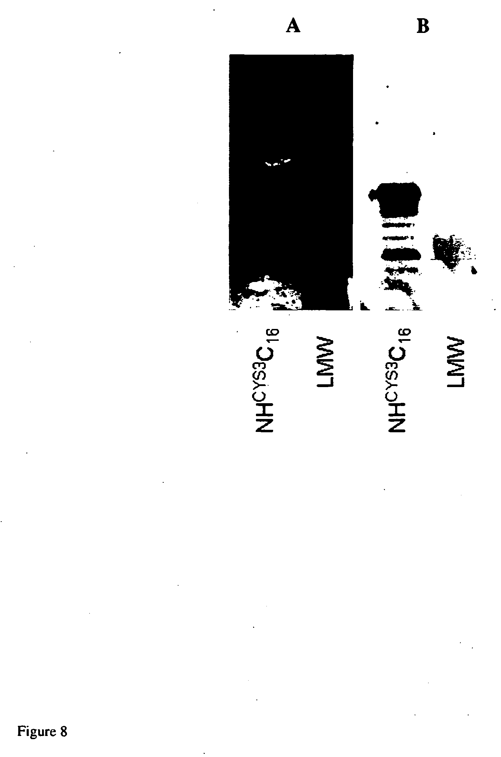

Modification of synthetic spider silks by a cysteine or lysine should allow for specific coupling of drugs, metals, polypeptides, quantum dots etc. In order to demonstrate the coupling of the SH-group of a cysteine to small organic molecules, the fluorophore rhodamine was coupled to the protein NH^{CYS3}C₁₆. Rhodamine was used in a maleimide-conjugated form, which reacts readily and very specifically with SH-groups at pH 7.0-7.5. Effective coupling was visualized by SDS-PAGE followed by fluorescence imaging and silver staining (Fig. 8).

### REFERENCES

1. Sambrook, J. and Russell, D. (2001) Molecular cloning*.*
2. Kroll, D. J., Abdel-Malek Abdel-Hafiz, H., Marcell, T., Simpson, S., Chen, C. Y., Gutierrez-Hartmann, A., Lustbader, J. W., and Hoeffler, J. P. (1993) A multifunctional prokaryotic protein expression system: overproduction, affinity purification, and selective detection, DNA Cell. Biol. 12, 441-453.
3. Gill, S. C. and von Hippel, P. H. (1989) Calculation of Protein Extinction Coefficients from Amino-Acid Sequence Data, Analytical Biochemistry 182, 319-326.
4. Blattner, F. R., Plunkett, G., Ill, Bloch, C. A., Perna, N. T., Burland, V., Riley, M., Collado-Vides, J., Glasner, J. D., Rode, C. K., Mayhew, G. F., Gregor, J., Davis, N. W., Kirkpatrick, H. A., Goeden, M. A., Rose, D. J., Mau, B., and Shao, Y. (1997) The complete genome sequence of Escherichia coli K-12, Science 277, 1453-1474.
5. Slotta, U., Tammer, M., Kremer, F., Koelsch, P., Scheibel, T. (2006) Structural analysis of spider silk films, Supromolecular chemistry 18, 465-471

## Claims

1. A modified spider silk protein comprising
(i) only one modified spider silk protein module according to SEQ ID NO: 1, SEQ ID NO: 3 or SEQ ID NO: 5 to SEQ ID NO: 7, or a variant thereof which differs in each case from said sequence by 1 amino acid substitution, 1 to 2 amino acid deletion(s), and/or 1 to 2 amino acid insertion(s), wherein one serine or glutamate in said spider silk protein module or variant thereof is replaced by one cysteine or lysine,
and wherein optionally an amino terminal TAG according to SEQ ID NO: 20 to SEQ ID NO: 24 containing one lysine or cysteine among other amino acids, or a carboxyl terminal TAG according to SEQ ID NO: 25 to SEQ ID NO: 28 containing one lysine or cysteine among other amino acids is added to said module or variant thereof, or
(ii) one spider silk protein module according to SEQ ID NO: 1 to SEQ ID NO: 7, or a variant thereof which differs in each case from said sequence by 1 amino acid substitution, 1 to 2 amino acid deletion(s), and/or 1 to 2 amino acid insertion(s), wherein an amino terminal TAG according to SEQ ID NO: 20 to SEQ ID NO: 24 containing one lysine or cysteine among other amino acids, or a carboxyl terminal TAG according to SEQ ID NO: 25 to SEQ ID NO: 28 containing one lysine or cysteine among other amino acids is added to said module or variant thereof, and
wherein the modified spider silk protein does not comprise any further lysine or cysteine residues.

2. The modified spider silk protein of claim 1, wherein other substances are coupled to the lysine or cysteine residues in the modified spider silk protein.

3. The modified spider silk protein of claim 2, wherein the other substances coupled to the lysine or cysteine residues are selected from the group consisting of polypeptides, polysaccharides, marker molecules, quantum dots, metals, nucleic acids, lipids and low molecular drugs.

4. The modified spider silk protein according to claims 1 to 3, wherein said spider silk protein comprises one spider silk module according to SEQ ID NO: 8 to 12 or SEQ ID NO: 14 to 19.

5. The modified spider silk protein of claims 1 to 4, wherein said spider silk protein comprises one or more further spider silk protein modules according to SEQ ID NO: 1 to SEQ ID NO: 7.

6. The modified spider silk protein of claim 5, wherein said spider silk protein comprises one or more of (AQ) and/or (QAQ) as spider silk protein modules, and preferably (AQ)₁₂, (AQ)₂₄, (QAQ)₈ or (QAQ)₁₆, wherein module A represents an amino acid sequence according to SEQ ID NO: 1 and module Q represents an amino acid sequence according to SEQ ID NO: 2.

7. The modified spider silk protein according to claim 5, wherein said spider silk protein comprises C₁₆ or C₃₂ as spider silk protein modules, wherein module C represents an amino acid sequence according to SEQ ID NO: 3.

8. The modified spider silk protein according to claim 5, wherein said spider silk protein comprises Y₈, Y₁₆, X₈, X₁₆, K₈, K₁₆ or Y₆X₂Sp₁K₂Y₂ as spider silk protein modules, wherein module Y represents an amino acid sequence according to SEQ ID NO: 7, module X represents an amino acid sequence according to SEQ ID NO: 6, K represents an amino acid sequence according to SEQ ID NO: 4, and sp represents an amino acid sequence according to SEQ ID NO: 5.

9. A nucleic acid sequence coding for a modified spider silk protein according to claims 1 to 8.

10. A vector, which comprises the nucleic acid sequence of claim 9.

11. The vector of claim 10, which further comprises one or more regulatory sequences, wherein the vector is an expression vector.

12. The vector of claim 11, wherein the vector is a plasmid or a viral vector, preferably a baculovirus system or a vaccinia virus vector system.

13. A host, which has been transformed with the vector of claims 10 to 12.

14. The host of claim 13, wherein the host is a prokaryotic cell, preferably *E. coli* or *Bacillus subtilis.*

15. The host of claim 13, wherein the host is a eukaryotic cell, preferably a mammalian cell, plant cell, yeast cell or an insect cell.

16. The host cell of claim 15, wherein
(i) the mammalian cell is a CHO, COS, HeLa, 293T, HEH or BHK cell,
(ii) the yeast cell is *Saccharomyces cerevisiae, Schizosaccharomyces pombe, Pichia pastoris, Candida albicans*, or *Hansenula polymorpha,*
(iii) the insect cell is selected from *Lepidoptera* insect cells, preferably from *Spodoptera frugiperda* and from *Trichoplusia ni* and more preferably is a Sf9, Sf21 or high five cell, and
(iv) the plant cell is derived from tobacco, potato, corn, pea and tomato.

17. A pharmaceutical or cosmetical composition containing a modified spider silk protein of claims 1 to 8 and a pharmaceutically acceptable carrier.

18. A fibre/thread, filament, film, foam, sphere, nanofibril, or hydrogel made from the modified spider silk protein of claims 1 to 8, or a paper, textile or leather product comprising a modified spider silk protein of claims 1 to 8 or a gel or foam comprising or consisting of a modified spider silk protein of claims 1 to 8.

19. The paper product, textile or leather product of claim 18, wherein the recombinant spider silk protein is present as a coating.

## Patentansprüche

1. Modifiziertes Spinnenseidenprotein umfassend
(i) nur ein modifiziertes Spinnenseidenproteinmodul gemäß SEQ ID NO: 1, SEQ ID NO: 3 oder SEQ ID NO: 5 bis SEQ ID NO: 7, oder eine Variante davon, welche sich jeweils von der besagten Sequenz durch 1 Aminosäuresubstitution, durch 1 bis 2 Aminosäuredeletion(en), und/oder durch 1 bis 2 Aminosäureinsertion(en) unterscheidet, wobei ein Serin oder eine Glutaminsäure in dem besagten Spinnenseidenproteinmodul oder in der Variante davon durch ein Cystein oder Lysin ersetzt ist,
und wobei optional ein aminoterminaler TAG gemäß SEQ ID NO: 20 bis SEQ ID NO: 24, welcher ein Lysin oder Cystein neben anderen Aminosäuren umfasst, oder ein carboxyterminaler TAG gemäß SEQ ID NO: 25 bis SEQ ID NO: 28, welcher ein Lysin oder Cystein neben anderen Aminosäuren umfasst, dem besagten Modul oder der Variante davon hinzugefügt ist, oder
(ii) ein Spinnenseidenproteinmodul gemäß SEQ ID NO: 1 bis SEQ ID NO: 7, oder einer Variante davon, welche sich jeweils von der besagten Sequenz durch 1 Aminosäuresubstitution, durch 1 bis 2 Aminosäuredeletion(en), und/oder durch 1 bis 2 Aminosäureinsertion(en) unterscheidet, wobei ein aminoterminaler TAG gemäß SEQ ID NO: 20 bis SEQ ID NO: 24, welcher ein Lysin oder Cystein neben anderen Aminosäuren umfasst, oder ein carboxyterminaler TAG gemäß SEQ ID NO: 25 bis SEQ ID NO: 28, welcher ein Lysin oder Cystein neben anderen Aminosäuren umfasst, dem besagten Modul oder der Variante davon hinzugefügt ist, und
wobei das modifizierte Spinnenseidenprotein keine weiteren Lysin- oder Cysteinreste umfasst.

2. Modifiziertes Spinnenseidenprotein gemäß Anspruch 1, wobei andere Substanzen an die Lysin- oder Cysteinreste in dem modifizierten Spinnenseidenprotein gekoppelt sind.

3. Modifiziertes Spinnenseidenprotein gemäß Anspruch 2, wobei die anderen Substanzen, die an die Lysin- oder Cysteinreste gekoppelt sind, aus der Gruppe bestehend aus Polypeptiden, Polysacchariden, Markermolekülen, Quantum-Dots, Metallen, Nukleinsäuren, Lipiden und niedrigmolekularen Wirkstoffen ausgewählt sind.

4. Modifiziertes Spinnenseidenprotein gemäß den Ansprüchen 1 bis 3, wobei das besagte Spinnenseidenprotein ein Spinnenseidenmodul gemäß SEQ ID NO: 8 bis 12 oder SEQ ID NO: 14 bis 19 umfasst.

5. Modifiziertes Spinnenseidenprotein nach den Ansprüchen 1 bis 4, wobei das besagte Spinnenseidenprotein ein oder mehrere weitere Spinnenseidenproteinmodule gemäß SEQ ID NO: 1 bis SEQ ID NO: 7 umfasst.

6. Modifiziertes Spinnenseidenprotein nach Anspruch 5, wobei das besagte Spinnenseidenprotein ein oder mehrere (AQ) und/oder (QAQ) als Spinnenseidenproteinmodule umfasst und vorzugsweise (AQ)₁₂, (AQ)₂₄, (QAQ)₈ oder (QAQ)₁₆ umfasst, wobei Modul A eine Aminosäuresequenz gemäß SEQ ID NO: 1 repräsentiert und Modul Q eine Aminosäuresequenz gemäß SEQ ID NO: 2 repräsentiert.

7. Modifiziertes Spinnenseidenprotein gemäß Anspruch 5, wobei das besagte Spinnenseidenprotein C₁₆ oder C₃₂ als Spinnenseidenproteinmodule umfasst, wobei Modul C eine Aminosäuresequenz gemäß SEQ ID NO: 3 repräsentiert.

8. Modifiziertes Spinnenseidenprotein gemäß Anspruch 5, wobei das besagte Spinnenseidenprotein Y₈, Y₁₆, X₈, X₁₆, K₈, K₁₆ oder Y₆X₂Sp₁K₂Y₂ als Spinnenseidenproteinmodule umfasst, wobei Modul Y eine Aminosäuresequenz gemäß SEQ ID NO: 7 repräsentiert, Modul X eine Aminosäuresequenz gemäß SEQ ID NO: 6 repräsentiert, Modul K eine Aminosäuresequenz gemäß SEQ ID NO: 4 repräsentiert und sp eine Aminosäuresequenz gemäß SEQ ID NO: 5 repräsentiert.

9. Nukleinsäuresequenz, die für ein modifiziertes Spinnenseidenprotein gemäß den Ansprüchen 1 bis 8 kodiert.

10. Vektor, welcher die Nukleinsäuresequenz nach Anspruch 9 umfasst.

11. Vektor nach Anspruch 10, welcher ferner eine oder mehrere regulatorische Sequenzen umfasst, wobei der Vektor ein Expressionsvektor ist.

12. Der Vektor nach Anspruch 11, wobei der Vektor ein Plasmid oder ein viraler Vektor ist, vorzugsweise ein Baculovirus-System oder ein Kuhpocken-Vektor-System ist.

13. Wirt, welcher mit dem Vektor nach den Ansprüchen 10 bis 12 transformiert worden ist.

14. Wirt nach Anspruch 13, wobei der Wirt eine prokaryotische Zelle, vorzugsweise *E. coli* oder *Bacillus subtilis* ist.

15. Wirt nach Anspruch 13, wobei der Wirt eine eukaryotische Zelle, vorzugsweise eine Säugetierzelle, eine Pflanzenzelle, eine Hefezelle oder eine Insektenzelle ist.

16. Wirtszelle nach Anspruch 15, wobei
(i) die Säugetierzelle eine CHO-, COS-, HeLa-, 293T-, HEH- oder BHK-Zelle ist,
(ii) die Hefezelle eine *Saccharomyces cerevisiae, Schizosaccharomyces pombe, Pichia pastoris, Candida albicans* oder *Hansenula polymorpha* ist,
(iii) die Insektenzelle ausgewählt ist aus *Lepidoptera*-Insektenzellen, vorzugsweise aus *Spodoptera frugiperda* und aus *Trichoplusia ni* und bevorzugter eine Sf9-, Sf21- oder high-five-Zelle ist, und
(iv) die Pflanzenzelle von Tabak, Kartoffeln, Getreide, der Erbse oder der Tomate abgeleitet ist.

17. Pharmazeutische oder kosmetische Zusammensetzung, die ein modifiziertes Spinnenseidenprotein nach den Ansprüchen 1 bis 8 und einen pharmazeutisch verträglichen Träger umfasst.

18. Faser/Faden, Filament, Film, Schaum, Kügelchen, Nanofaser oder Hydrogel hergestellt aus dem modifizierten Spinnenseidenprotein nach den Ansprüchen 1 bis 8, oder Papierprodukt, Textilprodukt oder Lederprodukt, welches ein modifiziertes Spinnenseidenprotein nach den Ansprüchen 1 bis 8 umfasst, oder Gel oder Schaum umfassend oder bestehend aus einem modifizierten Spinnenseidenprotein nach den Ansprüchen 1 bis 8.

19. Papierprodukt, Textilprodukt oder Lederprodukt nach Anspruch 18, wobei das rekombinante Spinnenseidenprotein als Beschichtung vorhanden ist.

## Revendications

1. Protéine de soie d'araignée modifiée, comprenant
(i) seulement un module de protéine de soie d'araignée modifiée selon SEQ ID NO: 1, SEQ ID NO: 3, ou SEQ ID NO: 5 à SEQ ID NO: 7, ou une variante de celui-ci qui diffère dans chaque cas de ladite séquence par 1 substitution d'acide aminé, 1 à 2 délétion(s) d'acides aminés, et/ou 1 à 2 insertion(s) d'acides aminés, tandis qu'une sérine ou un glutamate dans ledit module de protéine de soie d'araignée ou dans la variante de celui-ci est remplacé par une cystéine ou une lysine,
et tandis que, en option, un TAG amino-terminal selon SEQ ID NO: 20 à SEQ ID NO: 24 contenant une lysine ou une cystéine parmi d'autres acides aminés, ou un TAG carboxyl-terminal selon SEQ ID NO: 25 à SEQ ID NO: 28 contenant une lysine ou une cystéine parmi d'autres acides aminés est ajouté audit module ou à ladite variante de celui-ci, ou
(ii) un module de protéine de soie d'araignée selon SEQ ID NO: 1 à SEQ ID NO: 7, ou une variante de celui-ci qui diffère dans chaque cas de ladite séquence par 1 substitution d'acide aminé, 1 à 2 délétion(s) d'acides aminés, et/ou 1 à 2 insertion(s) d'acides aminés, tandis qu'un TAG amino-terminal selon SEQ ID NO: 20 à SEQ ID NO: 24 contient une lysine ou une cystéine parmi d'autres acides aminés, ou un TAG carboxyl-terminal selon SEQ ID NO: 25 à SEQ ID NO: 28 contenant une lysine ou une cystéine parmi d'autres acides aminés est ajouté audit module ou à ladite variante de celui-ci, et
tandis que la protéine de soie d'araignée modifiée ne comprend pas d'autres résidus lysine ou cystéine.

2. Protéine de soie d'araignée modifiée selon la revendication 1, dans laquelle d'autres substances sont couplées aux résidus lysine ou cystéine dans la protéine de soie d'araignée modifiée.

3. Protéine de soie d'araignée modifiée selon la revendication 2, dans laquelle les autres substances couplées aux résidus lysine ou cystéine sont choisies dans le groupe consistant en polypeptides, polysaccharides, molécules marqueurs, points de quantum, métaux, acides nucléiques, lipides et médicaments de faible masse moléculaire.

4. Protéine de soie d'araignée modifiée selon les revendications 1 à 3, dans laquelle ladite protéine de soie d'araignée comprend un module de soie d'araignée selon SEQ ID NO: 8 à 12 ou SEQ ID NO: 14 à 19.

5. Protéine de soie d'araignée modifiée selon les revendications 1 à 4, dans laquelle ladite protéine de soie d'araignée comprend un ou plusieurs autres modules de protéine de soie d'araignée selon SEQ ID NO: 1 à SEQ ID NO: 7.

6. Protéine de soie d'araignée modifiée selon la revendication 5, dans laquelle ladite protéine de soie d'araignée comprend un ou plusieurs parmi (AQ) et/ou (QAQ) comme modules de protéine de soie d'araignée, et de préférence (AQ)₁₂, (AQ)₂₄, (QAQ)₈ ou (QAQ)₁₆, tandis que le module A représente une séquence d'acides aminés selon SEQ ID NO: 1 et le module Q représente une séquence d'acides aminés selon SEQ ID NO: 2.

7. Protéine de soie d'araignée modifiée selon la revendication 5, dans laquelle ladite protéine de soie d'araignée comprend C₁₆ ou C₃₂ comme modules de protéine de soie d'araignée, tandis que le module C représente une séquence d'acides aminés selon SEQ ID NO: 3.

8. Protéine de soie d'araignée modifiée selon la revendication 5, dans laquelle ladite protéine de soie d'araignée comprend un Y₈, Y₁₆, X₈, X₁₆, K₈, K₁₆ ou Y₆X₂Sp₁K₂Y₂ à titre de modules de protéine de soie d'araignée, tandis que le module Y représente une séquence d'acides aminés selon SEQ ID NO: 7, le module X représente une séquence d'acides aminés selon SEQ ID NO: 6, K représente une séquence d'acides aminés selon SEQ ID NO: 4, et sp représente une séquence d'acides aminés selon SEQ ID NO: 5.

9. Séquence d'acide nucléique codant pour une protéine de soie d'araignée modifiée selon les revendications 1 à 8.

10. Vecteur, qui comprend la séquence d'acide nucléique selon la revendication 9.

11. Vecteur selon la revendication 10, qui comprend en outre une ou plusieurs séquences révélatrices, tandis que le vecteur est un vecteur d'expression.

12. Vecteur selon la revendication 11, dans lequel le vecteur est un plasmide ou un vecteur viral, de préférence un système de baculovirus ou un système de vecteur de virus de la vaccine.

13. Hôte, qui a été transformé avec le vecteur selon les revendications 10 à 12.

14. Hôte selon la revendication 13, dans lequel l'hôte est une cellule procaryote, de préférence *E. coli* ou *Bacillus subtilis.*

15. Hôte selon la revendication 13, dans lequel l'hôte est une cellule eucaryote, de préférence une cellule mammalienne, une cellule végétale, une cellule de levure ou une cellule d'insecte.

16. Cellule hôte selon la revendication 15, dans laquelle
(i) la cellule mammalienne est une cellule CHO, COS, HeLa, 293T, HEH ou BHK,
(ii) la cellule de levure est *Saccharomyces cerevisiae, Schizosaccharomyces pombe, Pichia pastoris, Candida albicans,* ou *Hansenula polymorpha,*
(iii) la cellule d'insecte est choisie parmi les cellules d'insectes de *Lepidoptera,* de préférence de *Spodoptera frugiperda* et de *Trichoplusia ni,* et plus préférablement est une cellule Sf9, Sf21 ou une cellule "high five", et
(iv) la cellule végétale est dérivée de tabac, pomme de terre, blé, pois et tomate.

17. Composition pharmaceutique ou cosmétique contenant une protéine de soie d'araignée modifiée selon les revendications 1 à 8 et un support pharmaceutiquement acceptable.

18. Fibre/fil, filament, pellicule, matière alvéolaire, sphère, nanofibrille, ou hydrogel réalisé à partir de la protéine de soie d'araignée modifiée selon les revendications 1 à 8, ou produit en papier, en textile ou en cuir comprenant une protéine de soie d'araignée modifiée selon les revendications 1 à 8, ou gel ou matière alvéolaire comprenant ou consistant en une protéine de soie d'araignée modifiée selon les revendications 1 à 8.

19. Produit en papier, en textile ou en cuir selon la revendication 18, dans lequel la protéine de soie d'araignée recombinante est présente sous la forme d'un revêtement.


### SEQUENCE LISTING

<110> Technische Universität München
<120> Modified spider silk proteins
<130> P21229
<150> US60/712,095
   <151> 2005-08-29
<160> 34
<170> PatentIn version 3.3
<210> 1
   <211> 24
   <212> PRT
   <213> Artificial
<220>
   <223> Modul A
<400> 1
   Gly Pro Tyr Gly Pro Gly Ala Ser Ala Ala Ala Ala Ala Ala Gly Gly
   1               5                   10                  15
   Tyr Gly Pro Gly Ser Gly Gln Gln
               20
<210> 2
   <211> 20
   <212> PRT
   <213> Artificial
<220>
   <223> Modul Q
<400> 2
   Gly Pro Gly Gln Gln Gly Pro Gly Gln Gln Gly Pro Gly Gln Gln Gly
   1               5                   10                  15
   Pro Gly Gln Gln
               20
<210> 3
   <211> 35
   <212> PRT
   <213> Artificial
<220>
   <223> Modul C
<400> 3
   Gly Ser Ser Ala Ala Ala Ala Ala Ala Ala Ala Ser Gly Pro Gly Gly
   1               5                   10                  15
   Tyr Gly Pro Glu Asn Gln Gly Pro Ser Gly Pro Gly Gly Tyr Gly Pro
               20                  25                  30
   Gly Gly Pro
           35
<210> 4
   <211> 27
   <212> PRT
   <213> Artificial
<220>
   <223> Modul K
<400> 4
   Gly Pro Gly Gly Ala Gly Gly Pro Tyr Gly Pro Gly Gly Ala Gly Gly
   1               5                   10                  15
   Pro Tyr Gly Pro Gly Gly Ala Gly Gly Pro Tyr
               20                  25
<210> 5
   <211> 28
   <212> PRT
   <213> Artificial
<220>
   <223> Modul sp
<400> 5
   Gly Gly Thr Thr Ile Ile Glu Asp Leu Asp Ile Thr Ile Asp Gly Ala
   1               5                   10                  15
   Asp Gly Pro Ile Thr Ile Ser Glu Glu Leu Thr Ile
               20                  25
<210> 6
   <211> 18
   <212> PRT
   <213> Artificial
<220>
   <223> Modul X
<400> 6
   Gly Gly Ala Gly Gly Ala Gly Gly Ala Gly Gly Ser Gly Gly Ala Gly
   1               5                   10                  15
   Gly Ser
<210> 7
   <211> 30
   <212> PRT
   <213> Artificial
<220>
   <223> Modul Y
<400> 7
   Gly Pro Gly Gly Ala Gly Pro Gly Gly Tyr Gly Pro Gly Gly Ser Gly
   1 5 10 15
   Pro Gly Gly Tyr Gly Pro Gly Gly Ser Gly Pro Gly Gly Tyr
   20 25 30
<210> 8
   <211> 24
   <212> PRT
   <213> Artificial
<220>
   <223> Modul AC
<400> 8
   Gly Pro Tyr Gly Pro Gly Ala Ser Ala Ala Ala Ala Ala Ala Gly Gly
   1 5 10 15
   Tyr Gly Pro Gly Cys Gly Gln Gln
   20
<210> 9
   <211> 24
   <212> PRT
   <213> Artificial
<220>
   <223> Modul AK
<400> 9
   Gly Pro Tyr Gly Pro Gly Ala Ser Ala Ala Ala Ala Ala Ala Gly Gly
   1 5 10 15
   Tyr Gly Pro Gly Lys Gly Gln Gln
   20
<210> 10
   <211> 35
   <212> PRT
   <213> Artificial
<220>
   <223> Modul CC
<400> 10
   Gly Ser Ser Ala Ala Ala Ala Ala Ala Ala Ala Ser Gly Pro Gly Gly
   1               5                   10                  15
   Tyr Gly Pro Glu Asn Gln Gly Pro Cys Gly Pro Gly Gly Tyr Gly Pro
               20                  25                  30
   Gly Gly Pro
           35
<210> 11
   <211> 35
   <212> PRT
   <213> Artificial
<220>
   <223> Modul CK1
<400> 11
   Gly Ser Ser Ala Ala Ala Ala Ala Ala Ala Ala Ser Gly Pro Gly Gly
   1               5                   10                  15
   Tyr Gly Pro Glu Asn Gln Gly Pro Lys Gly Pro Gly Gly Tyr Gly Pro
               20                  25                  30
   Gly Gly Pro
           35
<210> 12
   <211> 35
   <212> PRT
   <213> Artificial
<220>
   <223> Modul CK2
<400> 12
   Gly Ser Ser Ala Ala Ala Ala Ala Ala Ala Ala Ser Gly Pro Gly Gly
   1               5                   10                  15
   Tyr Gly Pro Lys Asn Gln Gly Pro Ser Gly Pro Gly Gly Tyr Gly Pro
               20                  25                  30
   Gly Gly Pro
           35
<210> 13
   <211> 35
   <212> PRT
   <213> Artificial
<220>
   <223> Modul CKC
<400> 13
   Gly Ser Ser Ala Ala Ala Ala Ala Ala Ala Ala Ser Gly Pro Gly Gly
   1               5                   10                  15
   Tyr Gly Pro Lys Asn Gln Gly Pro Cys Gly Pro Gly Gly Tyr Gly Pro
               20                  25                  30
   Gly Gly Pro
           35
<210> 14
   <211> 28
   <212> PRT
   <213> Artificial
<220>
   <223> Modul spC
<400> 14
   Gly Gly Thr Thr Ile Ile Glu Asp Leu Asp Ile Thr Ile Asp Gly Ala
   1               5                   10                  15
   Asp Gly Pro Ile Thr Ile Cys Glu Glu Leu Thr Ile
               20                  25
<210> 15
   <211> 28
   <212> PRT
   <213> Artificial
<220>
   <223> Modul spK
<400> 15
   Gly Gly Thr Thr Ile Ile Glu Asp Leu Asp Ile Thr Ile Asp Gly Ala
   1               5                   10                  15
   Asp Gly Pro Ile Thr Ile Lys Glu Glu Leu Thr Ile
               20                  25
<210> 16
   <211> 18
   <212> PRT
   <213> Artificial
<220>
   <223> Modul XC
<400> 16
   Gly Gly Ala Gly Gly Ala Gly Gly Ala Gly Gly Cys Gly Gly Ala Gly
   1 5 10 15
   Gly Ser
<210> 17
   <211> 18
   <212> PRT
   <213> Artificial
<220>
   <223> Modul XK
<400> 17
   Gly Gly Ala Gly Gly Ala Gly Gly Ala Gly Gly Lys Gly Gly Ala Gly
   1 5 10 15
   Gly Ser
<210> 18
   <211> 30
   <212> PRT
   <213> Artificial
<220>
   <223> Modul YC
<400> 18
   Gly Pro Gly Gly Ala Gly Pro Gly Gly Tyr Gly Pro Gly Gly Ser Gly
   1 5 10 15
   Pro Gly Gly Tyr Gly Pro Gly Gly Cys Gly Pro Gly Gly Tyr
   20 25 30
<210> 19
   <211> 30
   <212> PRT
   <213> Artificial
<220>
   <223> Modul YK
<400> 19
   Gly Pro Gly Gly Ala Gly Pro Gly Gly Tyr Gly Pro Gly Gly Ser Gly
   1 5 10 15
   Pro Gly Gly Tyr Gly Pro Gly Gly Lys Gly Pro Gly Gly Tyr
   20 25 30
<210> 20
   <211> 13
   <212> PRT
   <213> Artificial
<220>
   <223> Modul NHCYS1
<400> 20
   Gly Cys Gly Gly Gly Gly Gly Gly Ser Gly Gly Gly Gly
   1               5                   10
<210> 21
   <211> 8
   <212> PRT
   <213> Artificial
<220>
   <223> Modul NHCYS2
<400> 21
   Gly Cys Gly Gly Gly Gly Gly Gly
   1               5
<210> 22
   <211> 14
   <212> PRT
   <213> Artificial
<220>
   <223> Modul NHCYS3
<400> 22
   Gly Cys Gly Gly Ser Gly Gly Gly Gly Ser Gly Gly Gly Gly
   1               5                   10
<210> 23
   <211> 13
   <212> PRT
   <213> Artificial
<220>
   <223> Modul NHLYS1
<400> 23
   Gly Lys Gly Gly Gly Gly Gly Gly Ser Gly Gly Gly Gly
   1               5                   10
<210> 24
   <211> 8
   <212> PRT
   <213> Artificial
<220>
   <223> Modul NHLYS2
<400> 24
   Gly Lys Gly Gly Gly Gly Gly Gly
   1 5
<210> 25
   <211> 14
   <212> PRT
   <213> Artificial
<220>
   <223> Modul CHCYS1
<400> 25
   Gly Gly Gly Gly Ser Gly Gly Gly Gly Ser Gly Gly Cys Gly
   1 5 10
<210> 26
   <211> 9
   <212> PRT
   <213> Artificial
<220>
   <223> Modul CHCYS2
<400> 26
   Gly Gly Gly Gly Ser Gly Gly Cys Gly
   1 5
<210> 27
   <211> 14
   <212> PRT
   <213> Artificial
<220>
   <223> Modul CHLYS1
<400> 27
   Gly Gly Gly Gly Ser Gly Gly Gly Gly Ser Gly Gly Lys Gly
   1 5 10
<210> 28
   <211> 9
   <212> PRT
   <213> Artificial
<220>
   <223> Modul CHLYS2
<400> 28
   Gly Gly Gly Gly Ser Gly Gly Lys Gly
   1               5
<210> 29
   <211> 3238
   <212> DNA
   <213> Artificial
<220>
   <223> cloning vector pAZL
<400> 29
   tgtcgagaag tactagagga tcataatcag ccataccaca tttgtagagg ttttacttgc      60
   tttaaaaaac ctcccacacc tccccctgaa cctgaaacat aaaatgaatg caattgttgt    120
   tgttaacttg tttattgcag cttataatgg ttacaaataa agcaatagca tcacaaattt    180
   cacaaataaa gcattttttt cactgcattc tagttgtggt ttgtccaaac tcatcaatgt    240
   atcttatcat gtctggatct gatcactgct tgagcctagg agatccgaac cagataagtg    300
   aaatctagtt ccaaactatt ttgtcatttt taattttcgt attagcttac gacgctacac    360
   ccagttccca tctattttgt cactcttccc taaataatcc ttaaaaactc catttccacc    420
   cctcccagtt cccaactatt ttgtccgccc acagcggggc atttttcttc ctgttatgtt    480
   tttaatcaaa catcctgcca actccatgtg acaaaccgtc atcttcggct actttttctc    540
   tgtcacagaa tgaaaatttt tctgtcatct cttcgttatt aatgtttgta attgactgaa    600
   tatcaacgct tatttgcagc ctgaatggcg aatgggacgc gccctgtagc ggcgcattaa    660
   gcgcggcggg tgtggtggtt acgcgcagcg tgaccgctac acttgccagc gccctagcgc    720
   ccgctccttt cgctttcttc ccttcctttc tcgccacgtt cgccggcttt ccccgtcaag 780
   ctctaaatcg ggggctccct ttagggttcc gatttagtgc tttacggcac ctcgacccca 840
   aaaaacttga ttagggtgat ggttcacgta gtgggccatc gccctgatag acggtttttc 900
   gccctttgac gttggagtcc acgttcttta atagtggact cttgttccaa actggaacaa 960
   cactcaaccc tatctcggtc tattcttttg atttataagg gattttgccg atttcggcct 1020
   attggttaaa aaatgagctg atttaacaaa aatttaacgc gaattttaac aaaatattaa 1080
   cgtttacaat ttcaggtggc acttttcggg gaaatgtgcg cggaacccct atttgtttat 1140
   ttttctaaat acattcaaat atgtatccgc tcatgagaca ataaccctga taaatgcttc 1200
   aataatattg aaaaaggaag agtatgagta ttcaacattt ccgtgtcgcc cttattccct 1260
   tttttgcggc attttgcctt cctgtttttg ctcacccaga aacgctggtg aaagtaaaag 1320
   atgctgaaga tcagttgggt gcacgagtgg gttacatcga actggatctc aacagcggta 1380
   agatccttga gagttttcgc cccgaagaac gttttccaat gatgagcact tttaaagttc 1440
   tgctatgtgg cgcggtatta tcccgtattg acgccgggca agagcaactc ggtcgccgca 1500
   tacactattc tcagaatgac ttggttgagt actcaccagt cacagaaaag catcttacgg 1560
   atggcatgac agtaagagaa ttatgcagtg ctgccataac catgagtgat aacactgcgg 1620
   ccaacttact tctgacaacg atcggaggac cgaaggagct aaccgctttt ttgcacaaca 1680
   tgggggatca tgtaactcgc cttgatcgtt gggaaccgga gctgaatgaa gccataccaa 1740
   acgacgagcg tgacaccacg atgcctgtag caatggcaac aacgttgcgc aaactattaa 1800
   ctggcgaact acttactcta gcttcccggc aacaattaat agactggatg gaggcggata 1860
   aagttgcagg accacttctg cgctcggccc ttccggctgg ctggtttatt gctgataaat 1920
   ctggagccgg tgagcgtggg tctcgcggta tcattgcagc actggggcca gatggtaagc 1980
   cctcccgtat cgtagttatc tacacgacgg ggagtcaggc aactatggat gaacgaaata 2040
   gacagatcgc tgagataggt gcctcactga ttaagcattg gtaactgtca gaccaagttt 2100
   actcatatat actttagatt gatttaaaac ttcattttta atttaaaagg atctaggtga 2160
   agatcctttt tgataatctc atgaccaaaa tcccttaacg tgagttttcg ttccactgag 2220
   cgtcagaccc cgtagaaaag atcaaaggat cttcttgaga tccttttttt ctgcgcgtaa 2280
   tctgctgctt gcaaacaaaa aaaccaccgc taccagcggt ggtttgtttg ccggatcaag 2340
   agctaccaac tctttttccg aaggtaactg gcttcagcag agcgcagata ccaaatactg 2400
   tccttctagt gtagccgtag ttaggccacc acttcaagaa ctctgtagca ccgcctacat 2460
   acctcgctct gctaatcctg ttaccagtgg ctgctgccag tggcgataag tcgtgtctta 2520
   ccgggttgga ctcaagacga tagttaccgg ataaggcgca gcggtcgggc tgaacggggg 2580
   gttcgtgcac acagcccagc ttggagcgaa cgacctacac cgaactgaga tacctacagc 2640
   gtgagcattg agaaagcgcc acgcttcccg aagggagaaa ggcggacagg tatccggtaa 2700
   gcggcagggt cggaacagga gagcgcacga gggagcttcc agggggaaac gcctggtatc 2760
   tttatagtcc tgtcgggttt cgccacctct gacttgagcg tcgatttttg tgatgctcgt 2820
   caggggggcg gagcctatgg aaaaacgcca gcaacgcggc ctttttacgg ttcctggcct 2880
   tttgctggcc ttttgctcac atgttctttc ctgcgttatc ccctgattct gtggataacc 2940
   gtattaccgc ctttgagtga gctgataccg ctcgccgcag ccgaacgacc gagcgcagcg 3000
   agtcagtgag cgaggaagcg gaagagcgcc tgatgcggta ttttctcctt acgcatctgt 3060
   gcggtatttc acaccgcaga ccagccgcgt aacctggcaa aatcggttac ggttgagtaa 3120
   taaatggatg ccctgcgtaa gcgggtgtgg gcggacaata aagtcttaaa ctgaacaaaa 3180
   tagatcgagg aggatccatg ggacgaattc acggctaatg aaagcttact gcacagct 3238
<210> 30
   <211> 28
   <212> DNA
   <213> Artificial
<220>
   <223> primer pAZL-fwd
<400> 30
   cactgagcgt cagaccccgt agaaaaga 28
<210> 31
   <211> 63
   <212> DNA
   <213> Artificial
<220>
   <223> primer pAZLmut-rev
<400> 31
   ctcttaagct ttcattagcc tggaccacct ggaccgtagc cgcccgggcc gcaaggaccc 60
   tgg 63
<210> 32
   <211> 57
   <212> DNA
   <213> Artificial
<220>
   <223> synthetic oligonucleotide N1
<400> 32
   gatccatggg ttgcggtggc tctggtggtg gcgggtccgg aggcggtggc taatgaa 57
<210> 33
   <211> 57
   <212> DNA
   <213> Artificial
<220>
   <223> synthetic oligonucleotide N2
<400> 33
   agctttcatt agccaccgcc tccggacccg ccaccaccag agccaccgca acccatg 57
<210> 34
   <211> 12
   <212> PRT
   <213> Artificial
<220>
   <223> T7-tag
<400> 34
   Met Ala Ser Met Thr Gly Gly Gln Gln Met Gly Arg
   1               5                   10